# EUROPÄISCHE PATENTSCHRIFT

(11) **EP 3 392 040 B1**
(45) Veröffentlichungstag und Bekanntmachung des Hinweises auf die Patenterteilung: **19.01.2022**
(21) Anmeldenummer: 18177270.8
(22) Anmeldetag: 10.08.2016
(51) Int. Cl.: B41F 19/00, B41F 23/04, B41J 3/60, B41J 11/00, B41F 25/00, B41J 3/54

(54) **MASCHINENANORDNUNG MIT MEHREREN STATIONEN ZUM SEQUENTIELLEN BEARBEITEN BOGENFÖRMIGER SUBSTRATE**
MACHINE ARRANGEMENT HAVING A PLURALITY OF STATIONS FOR SEQUENTIALLY PROCESSING OF SHEET-TYPE SUBSTRATES
ENSEMBLE MACHINE À PLUSIEURS STATIONS POUR LE TRAITEMENT SÉQUENTIEL DE SUBSTRATS SOUS FORME DE FEUILLES

(30) Priorität: 09.09.2015 DE 102015217229; 29.04.2016 WO PCT/EP2016/059647
(43) Veröffentlichungstag der Anmeldung: 24.10.2018
(62) Teilanmeldung aus: 16750173.3
(73) Patentinhaber: Koenig & Bauer AG, 97080 Würzburg (DE)
(72) Erfinder: Ziegenbalg, Christian, 01689 Weinböhla (DE); Becker, Uwe, 01445 Radebeul (DE); Köhler, Ulrich, 01445 Radebeul (DE); Schumann, Frank, 01468 Moritzburg / Friedewald (DE); Reinsch, Carsten, 01445 Radebeul (DE); Koch, Heinz Michael, 01156 Dresden-Cossebaude (DE)
(74) Vertreter: Koenig & Bauer AG

(56) Entgegenhaltungen:
- WO-A1-2004/013704
- DE-A1- 10 312 870
- DE-A1-102009 000 518
- DE-A1-102012 200 650

## Beschreibung

Die Erfindung betrifft eine Maschinenanordnung zum sequentiellen Bearbeiten bogenförmiger Substrate gemäß Anspruch 1.

Durch die WO 2004/013704 A1 ist eine Digitaldruckmaschine für den direkten berührungslosen Bogendruck mit einem in Umfangsrichtung formatfreien Digitaldruckwerk bekannt, mit einer dem Digitaldruckwerk nachgeschalteten Transportvorrichtung, wobei die Transportvorrichtung an seinem Umfang Bogen haltende Greifer aufweist, wobei die die Transportvorrichtung vorzugsweise mehrere Transportzylinder und/oder Transportbänder und/oder Gegendruckzylinder aufweist.

Durch die EP 2 540 513 A1 ist eine Maschinenanordnung zum sequentiellen Bearbeiten mehrerer bogenförmiger jeweils eine Vorderseite und eine Rückseite aufweisender Substrate bekannt, aufweisend einen ersten Druckzylinder und einen zweiten Druckzylinder, wobei jeweils am Umfang des ersten Druckzylinders mindestens eine die Vorderseite des betreffenden Substrates bedruckende erste Non-Impact-Druckeinrichtung und in Drehrichtung des ersten Druckzylinders nach der ersten Non-Impact-Druckeinrichtung ein die von der ersten Non-Impact-Druckeinrichtung bedruckte Vorderseite des betreffenden Substrates trocknender Trockner angeordnet sind, wobei jeweils am Umfang des zweiten Druckzylinders mindestens eine die Rückseite des betreffenden Substrates bedruckende zweite Non-Impact-Druckeinrichtung und in Drehrichtung des zweiten Druckzylinders nach der zweiten Non-Impact-Druckeinrichtung ein die von der zweiten Non-Impact-Druckeinrichtung bedruckte Rückseite des betreffenden Substrates trocknender Trockner angeordnet sind, wobei der erste Druckzylinder und der zweite Druckzylinder einen gemeinsamen Walzenspalt bildend angeordnet sind, wobei der erste Druckzylinder in diesem gemeinsamen Walzenspalt das betreffende vorderseitig bedruckte und getrocknete Substrat unmittelbar an den zweiten Druckzylinder übergibt.

Durch die DE 103 12 870 A1 ist eine Digitaldruckmaschine für den Bogendruck bekannt, mit einem in Umfangsrichtung formatfreien Digitaldruckwerk, einem dem Digitaldruckwerk nachgeschalteten Zwischenzylinder, der mit einem elastischen Material zumindest teilweise überzogen ist, und einem dem Zwischenzylinder nachgeschalteten Gegendruckzylinder, wobei der Gegendruckzylinder Bogen haltende Greifer aufweist und der Zwischenzylinder an seinem Umfang die Greifer aufnehmende Aussparungen aufweist.

Durch die DE 10 2012 200 650 A1 ist eine als sogenannte Wendemaschine ausgebildete Bogendruckmaschine bekannt, wobei die dem beidseitigen Bedrucken von Druckbogen im sogenannten Schön- und Widerdruck dienende Bogendruckmaschine einen Bogenanleger, mehrere als Offset-Druckwerke ausgebildete Druckwerke, ein den Druckwerken nachgeschaltetes Lackwerk sowie einen dem Lackwerk nachgeschalteten Ausleger umfasst, wobei in die Bogendruckmaschine mindestens eine druckformlose Druckeinrichtung integriert ist, die vorzugsweise dem Bedrucken der Druckbogen mit einem dynamischen bzw. veränderlichen Druckbild dient.

Durch die DE 101 56 800 B4 ist ein Druckwerk bekannt, welches eine umlauffähig ausgebildete Transporteinrichtung zum Transportieren eines Bedruckstoffbogens, die mit einer Halteeinrichtung zum Halten des Bedruckstoffbogens ausgestattet ist, und einen auf die Transporteinrichtung ausgerichteten NIP(Non-Impact-Printing)-Druckkopf zum Bedrucken des Bedruckstoffbogens umfasst, wobei die Halteeinrichtung periodisch ins Innere der Transporteinrichtung verstellbar gelagert ist.

Durch die nachveröffentlichte DE 10 2015 211 440 B3 ist eine Bogentransporttrommel einer Bogen verarbeitenden Maschine mit mindestens zwei Bogenauflageflächen mit Saugöffnungen bekannt, wobei eine jeweilige Bogenauflagefläche aus einem ersten und einem zweiten Kammsegment mit Segmentzinken gebildet wird, wobei in den Segmentzinken Saugnuten und in der Bogenauflagefläche eines jeweiligen zweiten Kammsegments eine Hinterkantensaugnut vorgesehen ist und mit einem Drehventil zur getakteten Beaufschlagung mit Saugluft, wobei eine Mehrzahl von Unterdruckquellen vorgesehen ist und wobei durch das Drehventil eine Hinterkantensaugnut je einer Unterdruckquelle zugeordnet ist.

Durch die US 6 363 234 B2 ist eine Digitaldruckmaschine mit einem vierfachgroßen Druckzylinder bekannt.

Durch die US 7 909 454 B2 ist eine Digitaldruckmaschine bekannt, mit einem Druckzylinder, auf dessen Mantelfläche in Umfangsrichtung vier jeweils von einem Haltemittel gehaltene Druckbogen anordenbar sind, wobei die Druckbogen von einem einfachgroßen Transferzylinder an den Druckzylinder übergeben werden.

Durch die DE 10 2013 211 250 A1 ist eine Druckmaschine mit mehreren gemeinsam einen Bedruckstoff bedruckenden Druckwerken bekannt, wobei mindestens zwei dieser Druckwerke entlang eines für den Bedruckstoff vorgesehenen Transportweges in einer Reihe nacheinander angeordnet sind, wobei zumindest eines dieser in der Reihe angeordneten Druckwerke als ein Inkjetsystem ausgebildet ist, wobei das betreffende Inkjetsystem derart ausgebildet ist, dass dieses Inkjetsystem eine sich in einem laufenden Druckprozess verändernde oder zumindest veränderbare Information auf eine auf dem Bedruckstoff vorgesehene Zielfläche druckt, wobei mindestens eine auf dem Bedruckstoff angebrachte Marke vorhanden ist, wobei eine Erfassungseinrichtung zum Erfassen der mindestens einen auf dem Bedruckstoff angebrachten Marke vorgesehen ist, wobei zwischen dem betreffenden Inkjetsystem und einem der zu diesem Inkjetsystem in der Reihe angeordneten Druckwerke eine das betreffende Inkjetsystem in seiner Position zumindest quer zu dem für den Bedruckstoff vorgesehenen Transportweg einstellende Stelleinrichtung vorgesehen ist, wobei die aktuelle Position des betreffenden Inkjetsystems durch eine Positionierung des betreffenden Inkjetsystems mittels der Einstelleinrichtung anhand der mindestens einen in dem laufenden Druckprozess von der Erfassungseinrichtung erfassten Marke relativ zu einer Position der für den Druck der sich in dem laufenden Druckprozess verändernden oder zumindest veränderbaren Information vorgesehenen Zielfläche eingestellt oder zumindest einstellbar ist.

Durch die DE 10 2012 218 840 A1 ist ein Verfahren zum Transfer einer ablösbaren von bildgebenden Transferschicht von einer Transferfolie auf mit einer Kleberbeschichtung versehene Druckbogen mittels eines Beschichtungswerks bekannt, wobei das Beschichtungswerk einen gemeinsamen Transferspalt bildenden Gegendruckzylinder und eine Presswalze enthält, durch den die Transferfolie von einer Folienvorratsrolle die Presswalze berührend führbar ist, derart, dass sie mit der Transferschicht auf den auf dem Gegendruckzylinder geführten Druckbogen aufliegend und unter Druck zum Übertragen der Beschichtung gemeinsam mit dem Druckbogen durch den Transferspalt geführt wird, a) wobei in dem Auftragwerk ein bildmäßiger Kleberauftrag auf den Druckbogen erfolgt, b) wobei im Transferspalt unter Pressung ein Sujet der Transferschicht von der Transferfolie auf den mit einem Kleberbild versehenen Druckbogen übertragen wird, c) wobei wenigstens auf die derart auf den Druckbogen aufgetragene Transferschicht eine Schutzschicht aufgetragen wird, d) wobei nach dem Auftragen der Schutzschicht eine Trocknung der Schutzschicht auf dem Druckbogen erfolgt und e) wobei nach der Trocknung der Schutzschicht der Druckbogen unter Einbeziehung von Transferschicht und Schutzschicht in teil- oder ganzflächiger Ausdehnung bildhaft überdruckt wird.

Durch die DE 10 2014 010 904 B3 ist eine Vorrichtung zum beidseitigen Bedrucken von bogenförmigen Bedruckstoffen bekannt, wobei der Bedruckstoff auf einem Gegendruckzylinder um mehr als 360° geführt wird, wobei der Bedruckstoff mit der Widerdruckseite erneut in den Wirkbereich einer Farbauftragungseinheit gelangt, von welcher der Bedruckstoff auf einem vorgeordneten Gegendruckzylinder bereits auf der Schöndruckseite bedruckt wurde, wobei die Farbauftragungseinheit zwischen zwei einander nachgeordneten Gegendruckzylindern vorzugsweise verschwenkt werden kann, wobei die schwenkbare Farbauftragungseinheit z. B. ein Inkjet-Druckkopf ist.

Durch die DE 10 2009 000 518 A1 ist eine Bogendruckmaschine bekannt, mit einem Anleger zum Einschleusen zu bedruckender Druckbogen in die Bogendruckmaschine, mit mindestens einem Druckwerk und/oder Lackwerk zum Bedrucken der Druckbogen mit einem statischen, für alle Druckbogen identischen Druckbild, mit einem Ausleger zum Ausschleusen bedruckter Druckbogen aus der Bogendruckmaschine, und mit mindestens einer in die Bogendruckmaschine integrierten, druckformlosen Druckeinrichtung zum Bedrucken der Druckbogen mit einem insbesondere dynamischen, veränderlichen Druckbild, wobei die oder jede druckformlose Druckeinrichtung in der Bogendruckmaschine abhängig von Prozessparametern oder Betriebsparametern oder Auftragsparametern oder Qualitätsparametern ansteuerbar integriert ist.

Der Erfindung liegt die Aufgabe zugrunde, eine Maschinenanordnung zum sequentiellen Bearbeiten bogenförmiger Substrate zu schaffen.

Die Aufgabe wird erfindungsgemäß durch die Merkmale des Anspruches 1 gelöst. Die jeweils abhängigen Ansprüche zeigen vorteilhafte Ausgestaltungen und/oder Weiterbildungen der gefundenen Lösung.

Die mit der Erfindung erzielbaren Vorteile sind aus den nachfolgenden Erläuterungen ersichtlich.

Überdies ist die beschriebene Lösung in einer hybriden, bogenförmige Substrate bearbeitenden Maschinenanordnung verwendbar, vorzugsweise in einer hybriden Druckmaschine, die die hohe Produktivität einer konventionellen, z. B. in einem OffsetDruckverfahren oder in einem Flexodruckverfahren oder in einem Siebdruckverfahren druckenden Druckeinrichtung oder einer Beschichtungseinrichtung, insbesondere eines Lackwerkes variabel in Kombination mit mindestens einer flexibel jeweils veränderliche Druckbilder druckenden, z. B. als ein Tintenstrahldrucker ausgebildete Non-Impact-Druckeinrichtung nutzt, wobei sowohl die konventionelle Druckeinrichtung bzw. die Beschichtungseinrichtung als auch die Non-Impact-Druckeinrichtung in einer laufenden Produktion inline jeweils mit der für sie optimalen Arbeitsgeschwindigkeit verwendet werden. Eine solche hybride Maschinenanordnung ist insbesondere zur Produktion von Verpackungsmitteln, z. B. von Bogen zur Herstellung von Faltschachteln sehr vorteilhaft, weil jeweils die Stärken jeder der Druckeinrichtungen genutzt werden, was zu einer flexiblen und wirtschaftlichen Produktion der Verpackungsmittel führt. Ein Transport von bogenförmigen Substraten mittels Rotationskörpern, insbesondere Zylindern und Greiferleisten oder Greiferwagen jeweils mit einer Übergabe der bogenförmigen Substrate jeweils im Greiferschluss an eine nächstfolgende Bearbeitungsstation, wie dies von Bogenoffsetdruckmaschinen bekannt ist, stellt eine höchstmögliche Registergenauigkeit sicher.

Ausführungsbeispiele der Erfindung sind in den Zeichnungen dargestellt und werden im Folgenden näher beschrieben.

Es zeigen:
- Fig. 1: ein Blockschaltbild zur Darstellung von verschiedenen Produktionslinien;
- Fig. 2: eine erste Maschinenanordnung mit mehreren verschiedenen Bearbeitungsstationen;
- Fig. 3 bis 8: weitere Maschinenanordnungen jeweils mit mehreren verschiedenen Bearbeitungsstationen;
- Fig. 9: eine Maschinenanordnung zum beidseitigen sequentiellen Bearbeiten mehrerer bogenförmiger Substrate;
- Fig. 10: eine weitere Maschinenanordnung ohne Wendeeinrichtung zum beidseitigen sequentiellen Bearbeiten mehrerer bogenförmiger Substrate;
- Fig. 11: eine erfindungsgemäße Maschinenanordnung mit einer Wendeeinrichtung zum beidseitigen sequentiellen Bearbeiten mehrerer bogenförmiger Substrate;
- Fig. 12: eine Maschinenanordnung mit unterschiedlich langen Substratführungseinheiten;
- Fig. 13 bis 15: Maschinenanordnungen mit einem Druckzylinder und einer Transfertrommel verschiedenen Formats.

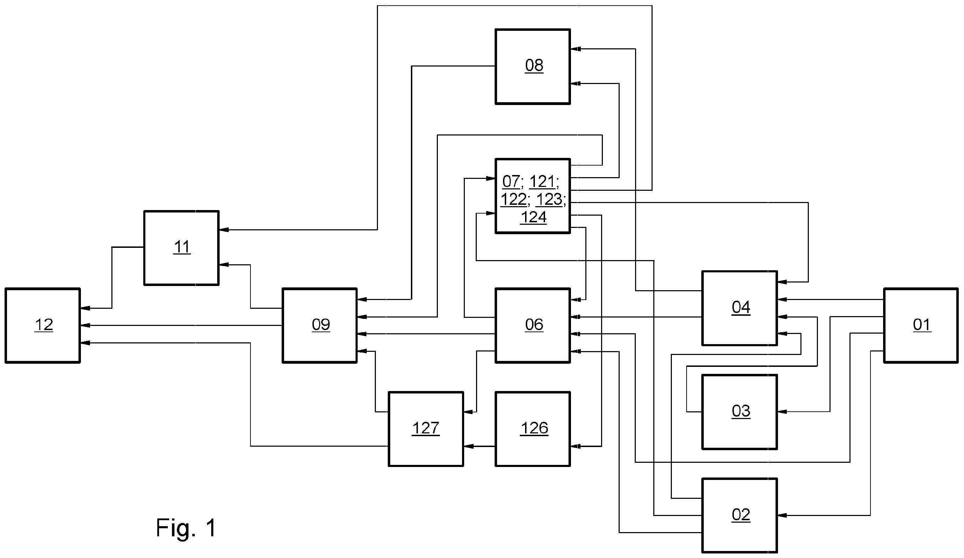

Fig. 1 veranschaulicht in einem Blockschaltbild verschiedene Produktionslinien, die jeweils mit einer Maschinenanordnung mit mehreren insbesondere verschiedenen Bearbeitungsstationen 01; 02; 03; 04; 06; 07; 08; 09; 11; 12 zur Bearbeitung mindestens eines bogenförmigen Substrates, insbesondere eines Bedruckstoffes, vorzugsweise eines insbesondere rechteckförmigen Druckbogens, kurz eines Bogens realisierbar sind, wobei dieses mindestens eine Substrat je nach Werkstoff, Materialstärke und/oder Grammatur biegesteif oder biegeschlaff ausgebildet ist. Dabei ist vorzugsweise jede dieser Bearbeitungsstationen 01; 02; 03; 04; 06; 07; 08; 09; 11; 12 jeweils als ein z. B. eigenständig funktionsfähiges Modul ausgebildet, wobei unter einem Modul eine i. d. R. eigenständig hergestellte oder zumindest eine für sich jeweils in einem eigenen Gestell montierte Maschineneinheit oder funktionelle Baugruppe verstanden werden soll. Jede der in der jeweiligen Maschinenanordnung angeordneten Bearbeitungsstationen 01; 02; 03; 04; 06; 07; 08; 09; 11; 12 wird also vorzugsweise eigenständig gefertigt und ist in einer bevorzugten Ausführung z. B. einzeln in seiner jeweiligen Funktion prüfbar. Die betreffende Maschinenanordnung, die jeweils durch eine Auswahl und Zusammenstellung von mindestens drei verschiedenen jeweils Bogen bearbeitenden, in einer bestimmten Produktion zusammenarbeitenden Bearbeitungsstationen 01; 02; 03; 04; 06; 07; 08; 09; 11; 12 gebildet ist, verkörpert jeweils eine bestimmte Produktionslinie. Jede der dargestellten Produktionslinien, die jeweils durch eine bestimmte Maschinenanordnung mit mehreren Bearbeitungsstationen 01; 02; 03; 04; 06; 07; 08; 09; 11; 12 verkörpert wird, ist jeweils insbesondere zur Produktion eines aus dem Bedruckstoff, vorzugsweise aus dem bedruckten Bogen gebildeten Verpackungsmittels ausgebildet. Die herzustellenden Verpackungsmitteln sind z. B. jeweils eine Faltschachtel, die jeweils aus bedruckten Bogen gefertigt werden. Die verschiedenen Produktionslinien sind daher insbesondere zur Produktion von unterschiedlichen Verpackungsmitteln ausgebildet. Dabei erfolgt die während einer bestimmten Produktion erforderliche Bearbeitung des Bedruckstoffes jeweils inline, d. h. die an der bestimmten Produktion beteiligten Bearbeitungsstationen 01; 02; 03; 04; 06; 07; 08; 09; 11; 12 werden beim Durchlauf des Bedruckstoffes durch die für die jeweilige Produktion gewählte, die jeweiligen Bearbeitungsstationen 01; 02; 03; 04; 06; 07; 08; 09; 11; 12 aufweisende Maschinenanordnung in einer geordneten Abfolge nacheinander und aufeinander abgestimmt zum Einsatz gebracht, ohne dass während der mit der jeweiligen Maschinenanordnung durchgeführten Produktion eine Zwischenlagerung für den Bedruckstoff, d. h. die bearbeiteten Bogen vorgesehen ist.

Allen in der Fig. 1 dargestellten Produktionslinien ist gemeinsam, dass sie jeweils mit einer Bearbeitungsstation 06 zusammenwirken, die mindestens eine Non-Impact-Druckeinrichtung 06, vorzugsweise mehrere, z. B. vier, fünf, sechs oder sieben insbesondere jeweils einzeln gesteuerte Non-Impact-Druckeinrichtungen 06 aufweist, wobei diese Non-Impact-Druckeinrichtungen 06 in Transportrichtung T des Bedruckstoffes vorzugsweise hintereinander angeordnet sind und derart ausgebildet sind, dass sie den Bedruckstoff jeweils zumindest nahezu in seiner vollständigen, quer zur Transportrichtung T gerichteten Breite bedrucken können. Eine Non-Impact-Druckeinrichtung 06 verwendet ein Druckverfahren ohne feste Druckform und kann prinzipiell von Druck zu Druck den Bedruckstoff, z. B. den jeweils dieser Druckeinrichtung 06 gerade zugeführten Bogen mit einem vom vorangegangenen Druckbild verschiedenen Druckbild bedrucken. Die jeweilige Non-Impact-Druckeinrichtung 06 ist jeweils insbesondere durch mindestens einen Tintenstrahldrucker oder durch mindestens einen Laserdrucker realisiert. Tintenstrahldrucker sind Matrixdrucker, bei denen durch den gezielten Abschuss oder das Ablenken kleiner Tintentröpfchen ein Druckbild erzeugt wird, wobei der Tintenstrahldrucker entweder als ein Gerät mit einem kontinuierlichen Tintenstrahl (Continuous Ink Jet = CIJ) oder als ein einzelne Tintentropfen verschießendes Gerät (Drop On Demand = DOD) ausgebildet ist. Laserdrucker erzeugen das jeweilige Druckbild in einem Elektrofotografieverfahren. Die Non-Impact-Druckeinrichtung 06 wird z. B. auch als eine Digitaldruckmaschine bezeichnet.

Im Folgenden wird beispielhaft davon ausgegangen, dass in der jeweiligen Maschinenanordnung mit mehreren Bearbeitungsstationen 01; 02; 03; 04; 06; 07; 08; 09; 11; 12 als Bedruckstoff jeweils eine Sequenz von insbesondere biegesteifen Bogen z. B. aus einem Papier, aus einem einlagigen oder mehrlagigen Karton oder aus einer Pappe insbesondere zu einem Verpackungsmittel verarbeitet wird. Die Bedruckstoffe Papier, Karton und Pappe unterscheiden sich in ihrem jeweiligen Flächengewicht, d. h. dem Gewicht in Gramm für einen Quadratmeter dieses Bedruckstoffes. Dabei gilt allgemein der vorgenannte Bedruckstoff mit einem Flächengewicht zwischen 7 g/m² und 150 g/m² als Papier, zwischen 150 g/m² und 600 g/m² als Karton und mit mehr als 600 g/m² als Pappe. Zur Herstellung von Faltschachteln werden insbesondere Kartone verwendet, die eine gute Bedruckbarkeit aufweisen und für eine anschließende Veredelung oder Verarbeitung wie z. B. für eine Lackierung und Stanzung geeignet sind. Diese Kartone sind von ihrem Faserstoffeinsatz z. B. holzfrei, leicht holzhaltig, holzhaltig oder altpapierhaltig. In ihrem Aufbau weisen mehrlagige Kartone eine Decklage, eine Einlage und als Rückseite eine Unterlage auf. Von ihrer Oberflächenbeschaffenheit her sind Kartone z. B. ungestrichen, pigmentiert, gestrichen oder gussgestrichen. Ein Format des Bogens liegt z. B. im Bereich zwischen 340 mm x 480 mm und 740 mm x 1060 mm, wobei bei den Formatangaben i. d. R. die erste Zahl eine Länge in Transportrichtung T der Bogen und die zweite Zahl eine orthogonal zur Transportrichtung T gerichtete Breite der Bogen angibt.

Im Blockschaltbild der Fig. 1 verläuft jede mit mehreren der Bearbeitungsstationen 01; 02; 03; 04; 06; 07; 08; 09; 11; 12 darstellbare Produktionslinie im Wesentlichen von rechts nach links, wobei jeder der jeweils zwei Bearbeitungsstationen 01; 02; 03; 04; 06; 07; 08; 09; 11; 12 miteinander verbindenden Richtungspfeile jeweils einen vom Bedruckstoff zu durchlaufenden Transportweg und die zugehörige Transportrichtung T andeutet, um von einer Bearbeitungsstation 01; 02; 03; 04; 06; 07; 08; 09; 11; 12 zur nächsten in der für die jeweilige Produktion bestimmten Maschinenanordnung ausgewählten Bearbeitungsstation 01; 02; 03; 04; 06; 07; 08; 09; 11; 12 zu gelangen. Jede Produktion beginnt mit in der Bearbeitungsstation 01 bereitgestellten Bogen, wobei die Bearbeitungsstation 01 als ein Anleger 01, z. B. als ein Bogenanleger 01 oder als ein Magazinanleger 01 ausgebildet ist. Ein Bogenanleger 01 nimmt i. d. R. einen z. B. auf einer Palette gestapelten Stapel von Bogen auf, wohingegen ein Magazinanleger 01 mehrere Fächer aufweist, in welche jeweils Bogen, insbesondere Stapel von z. B. verschiedenartigen Bogen oder Bogen verschiedener Formate eingelegt oder zumindest einlegbar sind. Der Anleger 01 vereinzelt z. B. mittels eines Saugkopfes 41 die gestapelten Bogen und führt diese in einer Sequenz von voneinander separierten Bogen oder in einem Schuppenstrom der in der bestimmten Produktion nächsten Bearbeitungsstation 02; 03; 04; 06 zu. Die nächste Bearbeitungsstation 02; 03; 04 ist z. B. als eine Primerauftrageinrichtung 02 oder als eine Kaltfolienauftrageinrichtung 03 oder als eine Offset-Druckeinrichtung 04 oder als eine Flexo-Druckeinrichtung 04 ausgebildet. Die nächste Bearbeitungsstation 06 kann auch direkt z. B. die mindestens eine Non-Impact-Druckeinrichtung 06 sein. Die Offset-Druckeinrichtung 04 ist vorzugsweise als eine Bogen-Offsetdruckmaschine ausgebildet, insbesondere als eine Bogendruckmaschine mit mehreren Druckwerken 86 in Reihenbauweise. Die Offset-Druckeinrichtung 04 versieht die Bogen mit mindestens einem statischen, d. h. während des Druckprozesses aufgrund der Gebundenheit an die verwendete Druckform unveränderlichen Druckbild, wohingegen die Non-Impact-Druckeinrichtung 06 die Bogen mit mindestens einem sich ändernden oder zumindest veränderlichen Druckbild versieht.

Falls die dem Anleger 01 nächste Bearbeitungsstation 03 die Kaltfolienauftrageinrichtung 03 ist, wird der Bogen anschließend von dort i. d. R. zur als Offset-Druckeinrichtung 04 ausgebildeten Bearbeitungsstation 04 transportiert. In der Kaltfolienauftrageinrichtung 03 wird eine von einer Trägerfolie abgelöste metallisierte Lackschicht auf den Bedruckstoff übertragen. Durch das Überdrucken dieser Lackschicht z. B. mit einer Offset-Druckeinrichtung 04 können unterschiedlichste Metalleffekte erzielt werden. Die Kaltfolienauftrageinrichtung 03 ist in vorteilhafter Weise z. B. in der Offset-Druckeinrichtung 04 integriert ausgebildet, indem zwei zusätzliche Druckwerke 87; 88 in der Offset-Druckeinrichtung 04 vorgesehen sind. Im in Transportrichtung T des Bedruckstoffes ersten Druckwerk 87 wird mittels einer Standarddruckform ein spezieller Klebstoff auf den Bedruckstoff, d. h. den jeweiligen Bogen aufgetragen. Ein in Transportrichtung T des Bedruckstoffes zweites Druckwerk 88 ist mit einer die zu übertragende Lackschicht aufweisenden Folientransfervorrichtung ausgestattet. Die die Lackschicht tragende Folie wird von einer Abwickelstation in einen Druckspalt zwischen einem Übertragungszylinder und einem mit diesem Übertragungszylinder zusammenwirkenden Druckzylinder geführt und mit dem Bedruckstoff in Kontakt gebracht. In der Lackschicht farbgebend sind eine Aluminiumschicht und eine Schutzlackschicht, deren Einfärbung den Farbeindruck beeinflusst. Durch Adhäsion einer Haftschicht mit der aufgedruckten Klebstoffschicht bleiben die Transferschichten auf dem Substrat haften. Die Trägerfolie wird anschließend wieder aufgewickelt. Nach dem Kaltfolientransfer ist inline, insbesondere in der Offset-Druckeinrichtung 04 das Überdrucken mit konventionellen Druckfarben sowie durch UV- und Hybridfarben möglich, um unterschiedliche metallische Farbtöne zu erzeugen.

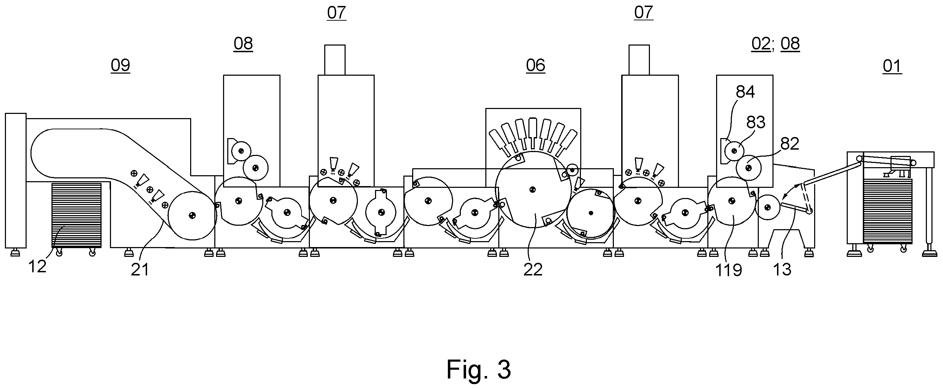

Ein z. B. besonders saugfähiger und/oder für ein Bedrucken mit einer Non-Impact-Druckeinrichtung 06 aufzubereitender Bedruckstoff wird vom Anleger 01 einer z. B. als eine Primerauftrageinrichtung 02 ausgebildeten nächsten Bearbeitungsstation 02 zugeführt, um zumindest eine Oberfläche dieses Bedruckstoffes vor einem Bedrucken oder Lackieren mit einem z. B. wasserbasierten Primer zu beschichten, insbesondere zu versiegeln. Das Primern stellt eine Grundierung oder Erstbeschichtung des Bedruckstoffes dar, um insbesondere eine Haftung einer danach auf den Bedruckstoff aufzutragenden Druckfarbe oder Tinte zu verbessern oder erst zu ermöglichen. Die Primerauftrageinrichtung 02 ist z. B. in Verbindung mit einem Druckwerk 86 einer Rotationsdruckmaschine ausgebildet und weist z. B. einen mit einem Anlagedruckzylinder 119 zusammenwirkenden Druckwerkszylinder 82 mit einer an diesen Druckwerkszylinder 82 angestellten oder zumindest anstellbaren Auftragswalze 83 vorzugsweise in Form einer Rasterwalze 83 auf sowie mindestens eine sich in Axialrichtung der Auftragswalze 83 erstreckende Rakel 84, insbesondere ein Kammerrakelsystem 84 (Fig. 3 bis 5, 8, 9). Der Primer wird mittels der Primerauftrageinrichtung 02 entweder vollflächig oder nur an bestimmten, d. h. zuvor festgelegten Stellen, d. h. partiell auf den Bedruckstoff aufgetragen. Der in der Primerauftrageinrichtung 02 bearbeitete Bedruckstoff, z. B. Bogen, wird als nächster Bearbeitungsstation z. B. einer Offset-Druckeinrichtung 04 und/oder z. B. einer Non-Impact-Druckeinrichtung 06 zugeführt.

Der von einer z. B. als eine Flexo-Druckeinrichtung 04 ausgebildeten Bearbeitungsstation 04 ausgeführte Flexodruck ist ein direktes Hochdruckverfahren, bei dem die erhabenen Stellen der Druckform bildtragend sind, welches häufig zum Bedrucken von Verpackungsmitteln aus Papier, Karton oder Pappe, aus metallisierter Folie oder aus einem Kunststoff wie z. B. PE, PET, PVC, PS, PP, PC verwendet wird. Im Flexodruck werden niedrigviskose Druckfarben und flexible Druckplatten verwendet, die aus Fotopolymer oder Gummi bestehen. Generell beinhaltet eine Flexo-Druckeinrichtung 04 a) eine Rasterwalze, über welche die Druckform eingefärbt wird, b) einen Druckzylinder, auch Formzylinder genannt, auf dem die Druckform befestigt ist, und c) einen Gegendruckzylinder, der den Bedruckstoff führt.

Die als Flexo-Druckeinrichtung 04 oder als Offset-Druckeinrichtung 04 ausgebildete, die Bogen jeweils mit mindestens einem statischen Druckbild bedruckende Bearbeitungsstation 04 weist vorzugsweise jeweils mehrere, z. B. mindestens vier Druckwerke 86 auf, wobei jedes Druckwerk 86 vorzugsweise eine andere Druckfarbe verdruckt, so dass der Bedruckstoff beim Durchgang durch die Flexo-Druckeinrichtung 04 oder die Offset-Druckeinrichtung 04 jeweils mehrfarbig, z. B. in einem Vierfarbendruck bedruckt wird. Als Druckfarben werden insbesondere die Farbtöne Gelb, Magenta, Cyan und Schwarz verwendet. In einer zum Flexodruckverfahren oder Offsetdruckverfahren alternativen Ausführung der Druckeinrichtung 04 ist die die Bogen jeweils mit mindestens einem statischen Druckbild bedruckende Bearbeitungsstation 04 als eine in einem Siebdruckverfahren druckende Druckeinrichtung 04 ausgebildet.

Nach einer Bearbeitung des Bedruckstoffes in der mindestens einen Non-Impact-Druckeinrichtung 06 wird dieser Bedruckstoff z. B. einer als ein Zwischentrockner 07 ausgebildeten Bearbeitungsstation 07 zugeführt, wobei dieser Zwischentrockner 07 als ein den betreffenden Bedruckstoff z. B. durch eine Bestrahlung mit infraroter oder ultravioletter Strahlung trocknend ausgebildet ist, wobei die Strahlungsart insbesondere davon abhängig ist, ob die auf den Bedruckstoff aufgetragene Druckfarbe oder Tinte wasserbasiert oder UV-härtend ist. Nach der Zwischentrocknung wird der Bedruckstoff z. B. einer als eine Lackiereinrichtung 08 ausgebildeten Bearbeitungsstation 08 zugeführt. Die Lackiereinrichtung 08 trägt auf dem Bedruckstoff z. B. einen Dispersionslack auf, wobei Dispersionslacke im Wesentlichen aus Wasser und Bindemitteln (Harzen) bestehen, wobei Tenside diese Dispersionen stabilisieren. Eine einen Dispersionslack auf den Bedruckstoff auftragende Lackiereinrichtung 08 besteht entweder aus einer Anilox-Walze, einem Kammerrakel und einer Auftragswalze (vergleichbar einem FlexoDruckwerk) oder aus einer Tauch- und Auftragswalze. Mittels einer Druckform vorzugsweise auf Fotopolymerisationsbasis werden z. B. flächige und/oder partielle Lackierungen aufgetragen. Verwendbar sind auch spezielle Lackplatten aus Gummi für vollflächige Lackierungen. Im Transportweg des Bedruckstoffes ist nach der Lackiereinrichtung 08 z. B. eine als ein Trockner 09 ausgebildete Bearbeitungsstation 09 angeordnet, wobei dieser Trockner 09 als ein den betreffenden Bedruckstoff durch eine Bestrahlung mit infraroter Strahlung oder durch Heißluft trocknend ausgebildet ist. Falls die betreffende Maschinenanordnung entlang des Transportweges des Bedruckstoffes mehrere Trockner 07; 09 aufweist, ist der Trockner mit dem Bezugszeichen 09 vorzugsweise der in Transportrichtung T des Bedruckstoffes letzte dieser mehreren Trockner 07; 09, wobei der oder die Zwischentrockner 07 und der (End-)Trockner 09 baulich gleich sind oder auch unterschiedlich ausgebildet sein können. Falls dem Trockner 09 ein durch ultraviolette Strahlung trocknender Bedruckstoff zugeführt wird, d. h. ein Bedruckstoff, auf dem eine durch eine UV-Strahlung härtende Druckfarbe oder Tinte oder ein durch eine UV-Strahlung härtender Lack, z. B. ein Glanzlack aufgetragen ist, ist dieser Trockner 09 mit einer ultraviolette Strahlung erzeugenden Strahlungsquelle ausgestattet. Mit Dispersionslacken lassen sich im Vergleich zum klassischen Öldrucklack intensivere Glanz- und Mattwirkungen erzielen. Spezielle optische Wirkungen können durch Effektpigmente im Lack erreicht werden. Die Primerauftrageinrichtung 02, die Kaltfolienauftrageinrichtung 03 und die Lackiereinrichtung 08 können unter dem Begriff Beschichtungseinrichtung 02; 03; 08 zusammengefasst werden.

Nach der Trocknung wird der Bedruckstoff z. B. einer Bearbeitungsstation 11 zugeführt, die am Bedruckstoff eine mechanische Weiterverarbeitung ausführt, z. B. durch Stanzen, Rillen und/oder Trennen von Teilen, insbesondere Ausbrechen von Nutzen aus ihrem jeweiligen Verbund im vorzugsweise bedruckten Bogen. Jede der vorgenannten Weiterverarbeitungen wird jeweils in bzw. von einem Bearbeitungswerk 46 ausgeführt. Die mechanische Weiterverarbeitung wird vorzugsweise im Zusammenwirken mit einem den jeweiligen Bogen transportierenden Zylinder ausgeführt. Danach oder direkt vom Trockner 09 gelangt der Bedruckstoff zu einer Auslage 12, welche in jeder der in der Fig. 1 dargestellten, jeweils durch eine bestimmte Anordnung von Bearbeitungsstationen 01; 02; 03; 04; 06; 07; 08; 09; 11; 12 verkörperten Produktionslinien jeweils die letzte Bearbeitungsstation 12 bildet. In der Auslage 12 werden die zuvor bearbeiteten Bogen z. B. auf einer Palette vorzugsweise gestapelt.

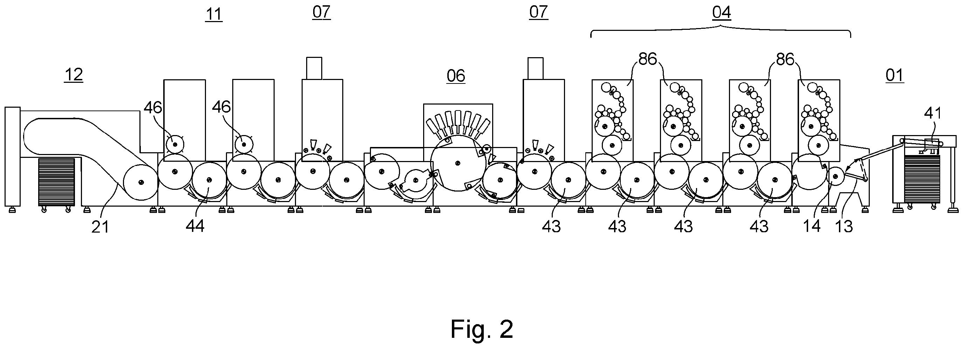

Wie in den Fig. 2 bis 8 dargestellt, ist die bisher erwähnte Reihenfolge der in der jeweiligen Maschinenanordnung angeordneten Bearbeitungsstationen 01; 02; 03; 04; 06; 07; 08; 09; 11; 12 nur beispielhaft und in Abhängigkeit von dem jeweils herzustellenden Druckprodukt modifizierbar.

In der Fig. 1 beispielhaft dargestellte, insbesondere für die Produktion von Verpackungsmitteln verwendete Produktionslinien weisen jeweils eine Maschinenanordnung mit einer Auswahl aus der Menge der zuvor genannten Bearbeitungsstationen 01; 02; 03; 04; 06; 07; 08; 09; 11; 12 auf. Es sind beispielsweise folgende Produktionslinien gebildet oder zumindest bildbar:
1. Bogenanleger 01; Primerauftrageinrichtung 02; Non-Impact-Druckeinrichtung 06; Zwischentrockner 07 mit IR-Strahlungsquelle für Dispersionslack; Lackiereinrichtung 08; Trockner 09 mit IR-Strahlungsquelle oder Heißluft; Auslage 12
2. Bogenanleger 01; Primerauftrageinrichtung 02; Non-Impact-Druckeinrichtung 06; Trockner 09 mit IR-Strahlungsquelle oder Heißluft; Auslage 12
3. Bogenanleger 01; Primerauftrageinrichtung 02; Non-Impact-Druckeinrichtung 06; Zwischentrockner 07 mit IR-Strahlungsquelle; Lackiereinrichtung 08 für Dispersionslack und UV-härtendem Lack; Trockner 09 mit IR-Strahlungsquelle oder Heißluft und mit UV-Strahlungsquelle; Auslage 12
4. Bogenanleger 01; Kaltfolienauftrageinrichtung 03; Offset-Druckeinrichtung 04; Non-Impact-Druckeinrichtung 06; Trockner 09 mit IR-Strahlungsquelle oder Heißluft; Auslage 12
5. Bogenanleger 01; Primerauftrageinrichtung 02; Non-Impact-Druckeinrichtung 06; Zwischentrockner 07 mit IR-Strahlungsquelle für Dispersionslack; Lackiereinrichtung 08; Trockner 09 mit IR-Strahlungsquelle oder Heißluft; mechanische Weiterverarbeitungseinrichtung 11; Auslage 12
6. Bogenanleger 01; Offset-Druckeinrichtung 04; Non-Impact-Druckeinrichtung 06; Zwischentrockner 07 mit IR-Strahlungsquelle; mechanische Weiterverarbeitungseinrichtung 11; Auslage 12
7. Bogenanleger 01; Non-Impact-Druckeinrichtung 06; Trockner 09 mit IR-Strahlungsquelle oder Heißluft; Auslage 12
8. Bogenanleger 01; Non-Impact-Druckeinrichtung 06; Zwischentrockner 07 mit UV-Strahlungsquelle; Trockner 09 mit UV-Strahlungsquelle; Auslage 12
9. Bogenanleger 01; Non-Impact-Druckeinrichtung 06; Zwischentrockner 07 mit UV-Strahlungsquelle; Trockner 09 mit UV-Strahlungsquelle; mechanische Weiterverarbeitungseinrichtung 11; Auslage 12
10. Bogenanleger 01; Non-Impact-Druckeinrichtung 06; Zwischentrockner 07 mit IR-Strahlungsquelle; Offset-Druckeinrichtung 04; Lackiereinrichtung 08; Trockner 09 mit IR-Strahlungsquelle oder Heißluft; Auslage 12
11. Magazinanleger 01; Primerauftrageinrichtung 02; Non-Impact-Druckeinrichtung 06; Zwischentrockner 07 mit IR-Strahlungsquelle; Lackiereinrichtung 08; Trockner 09 mit IR-Strahlungsquelle oder Heißluft; Auslage 12
12. Magazinanleger 01; Primerauftrageinrichtung 02; Non-Impact-Druckeinrichtung 06; Zwischentrockner 07 mit IR-Strahlungsquelle; Trockner 09 mit IR-Strahlungsquelle oder Heißluft; mechanische Weiterverarbeitungseinrichtung 11; Auslage 12
13. Magazinanleger 01; Non-Impact-Druckeinrichtung 06; Zwischentrockner 07 mit UV-Strahlungsquelle; Lackiereinrichtung 08; Trockner 09 mit UV-Strahlungsquelle; Auslage 12

Dabei ist mindestens eine der mit der mindestens einen Non-Impact-Druckeinrichtung 06 zusammenwirkenden Bearbeitungsstationen 01; 02; 03; 04; 07; 08; 09; 11; 12 zur Beteiligung an der Bearbeitung der Bogen jeweils in Abhängigkeit davon ausgewählt, ob die insbesondere mit der Non-Impact-Druckeinrichtung 06 auf den jeweiligen Bogen aufzutragende Druckfarbe als eine wasserbasierte Druckfarbe bzw. Tinte oder als eine durch ultraviolette Strahlung aushärtende Druckfarbe bzw. Tinte ausgebildet ist. Damit ist die jeweilige Maschinenanordnung die Bogen jeweils mit einer wasserbasierten Druckfarbe oder mit einer durch ultraviolette Strahlung aushärtenden Druckfarbe bedruckend ausgebildet.

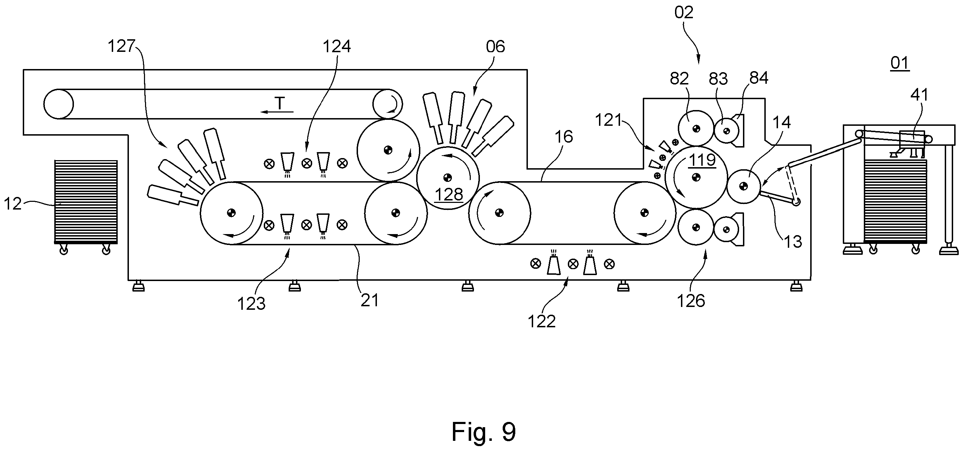

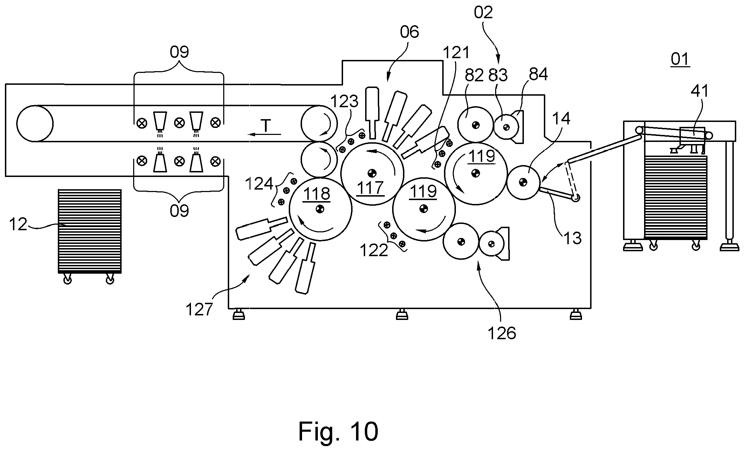

Weitere, i. V. m. den Fig. 9 und 10 näher erläuterte nicht erfindungsgemäße Maschinenanordnungen mit einer Auswahl aus der Menge der zuvor genannten Bearbeitungsstationen 01; 02; 03; 04; 06; 07; 08; 09; 11; 12 sehen z. B. Produktionslinien vor, die im Wesentlichen folgende Bearbeitungsstationen aufweisen: Bogenanleger 01; erste Primerauftrageinrichtung 02; erster Trockner 121; erste Non-Impact-Druckeinrichtung 06; zweiter Trockner 122; zweite Primerauftrageinrichtung 126; dritter Trockner 123; zweite Non-Impact-Druckeinrichtung 127; vierter Trockner 124; Auslage 12

Eine hier beispielhaft genannte vorteilhafte Maschinenanordnung weist mehrere Bearbeitungsstationen zur Bearbeitung von Bogen auf, wobei in Transportrichtung T der Bogen mehrere Bearbeitungsstationen 01; 02; 03; 04; 06; 07; 08; 09; 11; 12 nacheinander zur Inline-Bearbeitung dieser Bogen angeordnet sind, wobei wenigstens eine dieser Bearbeitungsstationen 06 als eine Non-Impact-Druckeinrichtung 06 ausgebildet ist, wobei eine in Transportrichtung T der Bogen der Non-Impact-Druckeinrichtung 06 vorgeordnete erste Bearbeitungsstation 01 als ein Bogenanleger 01 oder als ein Magazinanleger 01 ausgebildet ist, wobei eine zwischen der ersten Bearbeitungsstation 01 und der Non-Impact-Druckeinrichtung 06 angeordnete Bearbeitungsstation 08 als eine jeweils einen Lack auf die Bogen auftragende erste Beschichtungseinrichtung 08 ausgebildet ist, wobei zwischen der ersten Beschichtungseinrichtung 08 und der Non-Impact-Druckeinrichtung 06 ein erster Trockner 07 angeordnet ist, wobei ein erste Transportzylinderanordnung 17 die Bogen vom ersten Trockner 07 zur Non-Impact-Druckeinrichtung 06 transportierend angeordnet ist, wobei in Transportrichtung T der Bogen nach der Non-Impact-Druckeinrichtung 06 ein zweiter Trockner 07 angeordnet ist, wobei eine Einrichtung zur Übergabe der von der Non-Impact-Druckeinrichtung 06 kommenden Bogen an eine zweite Beschichtungseinrichtung 08 vorgesehen ist, wobei der zweiten Beschichtungseinrichtung 08 ein dritter Trockner 09 nachgeordnet ist, wobei in Transportrichtung T der Bogen nach dem dritten Trockner 09 eine Auslage 12 für die Bogen angeordnet ist. Dabei kann zwischen dem dritten Trockner 09 und der Auslage 12 zusätzlich noch eine mechanische Weiterverarbeitungseinrichtung 11 angeordnet sein. Ferner ist in Transportrichtung T der Bogen vor der Non-Impact-Druckeinrichtung 06 z. B. eine eine Kaltfolie auftragende Beschichtungseinrichtung 03 angeordnet. Die Non-Impact-Druckeinrichtung 06 weist entlang des Transportweges der Bogen vorzugsweise mehrere jeweils einzeln gesteuerte Tintenstrahldrucker auf. Im Wirkungsbereich der Non-Impact-Druckeinrichtung 06 sind die Bogen vorzugsweise jeweils auf einer Transporteinrichtung plan aufliegend geführt, wobei die Transporteinrichtung zumindest im Wirkungsbereich der Non-Impact-Druckeinrichtung 06 für die Bogen jeweils einen gekrümmten Transportweg aufweist, wobei die Transporteinrichtung im Wirkungsbereich der Non-Impact-Druckeinrichtung 06 als ein Druckzylinder 22 ausgebildet ist. In Transportrichtung T der Bogen ist vor der Non-Impact-Druckeinrichtung 06 z. B. eine Übergabeeinrichtung angeordnet, wobei die Übergabeeinrichtung die Bogen z. B. jeweils zumindest in ihrem Axialregister und/oder Umfangsregister registerhaltig relativ zur Druckposition der Non-Impact-Druckeinrichtung 06 ausrichtet, wobei die Übergabeeinrichtung z. B. eine den jeweiligen Bogen mittels Saugluft haltende Saugtrommel 32 aufweist. Diese Maschinenanordnung ist die Bogen insbesondere jeweils mit einer wasserbasierten Druckfarbe oder mit einer durch ultraviolette Strahlung aushärtenden Druckfarbe bedruckend ausgebildet. Diese Maschinenanordnung ist insbesondere unterschiedliche Verpackungsmittel produzierend ausgebildet. Die Einrichtung zur Übergabe der von der Non-Impact-Druckeinrichtung 06 kommenden Bogen an die zweite Beschichtungseinrichtung 08 ist z. B. als eine zweite Transportzylinderanordnung 19 ausgebildet.

Fig. 2 zeigt beispielhaft eine Maschinenanordnung mit mehreren Bearbeitungsstationen 01; 02; 03; 04; 06; 07; 08; 09; 11; 12 gemäß der vorstehend genannten Produktionslinie Nr. 6. Bogen werden in einem Bogenanleger 01 z. B. mit einem Saugkopf 41 einzeln von einem Stapel aufgegriffen und nacheinander in einem Takt von z. B. 10.000 Stück pro Stunde an eine Offset-Druckeinrichtung 04 mit z. B. vier in einer Reihe angeordneten Druckwerken 86 übergeben. Für eine Übergabe der Bogen von einem zum nächsten der in einer Reihe angeordneten Druckwerke 86 ist jeweils ein Rotationskörper, insbesondere ein Zylinder, vorzugsweise eine Transfertrommel 43 vorgesehen, die jeweils zwischen zwei unmittelbar benachbarten Druckwerken 86 angeordnet ist. Die Offset-Druckeinrichtung 04 übernimmt die ihr vom Bogenanleger 01 zugeführten Bogen z. B. mit einem Schwinggreifer 13 und leitet die Bogen an eine Übergabetrommel 14 der Offset-Druckeinrichtung 04, wobei die Bogen dann in der Offset-Druckeinrichtung 04 in einem Greiferschluss von einem zum nächsten Druckwerk 86 geführt werden. In der Offset-Druckeinrichtung 04 werden die Bogen zumindest einseitig bedruckt. Beim Vorhandensein einer Wendeeinrichtung 23 können die Bogen in der Offset-Druckeinrichtung 04 auch beidseitig bedruckt werden, d. h. im Schön- und Widerdruck. Nach dem Durchlauf durch die hier z. B. als Offset-Druckeinrichtung 04 ausgebildeten Bearbeitungsstation 04 wird der betreffende vorzugsweise vierfarbig bedruckte Bogen mittels der ersten Transportzylinderanordnung 17 an eine Non-Impact-Druckeinrichtung 06 übergeben. Die Non-Impact-Druckeinrichtung 06 weist vorzugsweise mehrere, z. B. fünf linear in einer Reihe angeordnete insbesondere jeweils einzeln gesteuerte Tintenstrahldrucker auf. Danach erfolgt eine Trocknung der in der Offset-Druckeinrichtung 04 mit mindestens einem statischen Druckbild und in der Non-Impact-Druckeinrichtung 06 mit mindestens einem sich ändernden oder zumindest veränderlichen Druckbild versehenen Bogen in einem Trockner 07 bzw. Zwischentrockner 07 vorzugsweise mit einer IR-Strahlungsquelle. Wiederum danach werden die Bogen in einer mechanischen Weiterverarbeitungseinrichtung 11 z. B. durch Stanzen und/oder Rillen und/oder Ausbrechen von Nutzen aus dem jeweiligen Bogen weiterverarbeitet. Letztlich werden die Bogen und/oder jeweils aus den Bogen gelöste Nutzen in einer Auslage 12 gesammelt, insbesondere gestapelt. Im Wirkungsbereich des ersten Greifersystems 16 bzw. des ersten Kettenförderers 16 kann jeweils entlang des für die Bogen vorgesehenen Transportweges eine Auslage 12, insbesondere eine Mehrstapelauslage vorgesehen sein. Ebenso ist in Transportrichtung T der Bogen z. B. nach der mechanischen Weiterverarbeitungseinrichtung 11 eine Mehrstapelauslage angeordnet.

Die im Anleger 01, insbesondere im Bogenanleger 01 von einem Stapel aufgegriffenen Bogen werden beabstandet voneinander einzeln durch die Offset-Druckeinrichtung 04 mit einer ersten Transportgeschwindigkeit transportiert. Die von der Offset-Druckeinrichtung 04 an die Non-Impact-Druckeinrichtung 06 übergebenen Bogen werden in dieser Non-Impact-Druckeinrichtung 06 mit einer zweiten Transportgeschwindigkeit transportiert, wobei die in der Non-Impact-Druckeinrichtung 06 geltende zweite Transportgeschwindigkeit i. d. R. geringer ist als die in der Offset-Druckeinrichtung 04 geltende erste Transportgeschwindigkeit. Zur Anpassung der in der Offset-Druckeinrichtung 04 geltenden ersten Transportgeschwindigkeit an die i. d. R. geringere in der Non-Impact-Druckeinrichtung 06 geltende zweite Transportgeschwindigkeit wird z. B. die zwischen direkt aufeinanderfolgenden Bogen bestehende Bogenlücke, d. h. der Abstand, der sich z. B. aufgrund einer Greiferkanalbreite für die im Greiferschluss durch die Offset-Druckeinrichtung 04 transportierten Bogen ergibt, bei der Übergabe dieser Bogen von der Offset-Druckeinrichtung 04 an die Non-Impact-Druckeinrichtung 06 vorzugsweise verringert, wobei eine solche Abstandsverringerung bezogen auf ihren ursprünglichen Abstand z. B. im Bereich zwischen 1% und 98% liegt. Damit werden direkt aufeinanderfolgende Bogen auch in der Non-Impact-Druckeinrichtung 06 voneinander beabstandet transportiert, jedoch mit einer i. d. R. geringeren Bogenlücke bzw. mit einem geringeren Abstand als in der Offset-Druckeinrichtung 04 und demzufolge auch mit einer geringeren zweiten Transportgeschwindigkeit. Diese zweite Transportgeschwindigkeit wird vorzugsweise beibehalten, wenn in der Non-Impact-Druckeinrichtung 06 bedruckte Bogen zunächst an einen Zwischentrockner 07 oder Trockner 09 und von dort z. B. mittels eines Anlegetisches an eine mechanische Weiterverarbeitungseinrichtung 11 weiter bis zur Auslage 12 transportiert werden. Jedoch können die Bogen von ihrer zweiten Transportgeschwindigkeit auch auf eine dritte Transportgeschwindigkeit gebracht werden, wenn dies z. B. die mechanische Weiterverarbeitungseinrichtung 11 erfordert, wobei die dritte Transportgeschwindigkeit i. d. R. höher ist als die zweite Transportgeschwindigkeit und z. B. wieder der insbesondere in der Offset-Druckeinrichtung 04 geltenden ersten Transportgeschwindigkeit entspricht. Vor der mechanischen Weiterverarbeitungseinrichtung 11 ist z. B. die zweite Transportzylinderanordnung 19 vorgesehen, welche die aus dem Zwischentrockner 07 oder Trockner 09 kommenden Bogen aufgreift und zur mechanischen Weiterverarbeitungseinrichtung 11 transportiert. Auch im Bereich der mechanischen, in Reihe z. B. mehrere Bearbeitungswerke 46 aufweisenden Weiterverarbeitungseinrichtung 11 ist für eine Übergabe der Bogen von einer zur nächsten der in einer Reihe angeordneten Bearbeitungswerke 46 jeweils ein Rotationskörper, insbesondere ein Zylinder, vorzugsweise eine Transfertrommel 44 vorgesehen, die jeweils zwischen zwei benachbarten Bearbeitungswerken 46 angeordnet ist. Eines der Bearbeitungswerke 46 ist z. B. als ein Stanzwerk, ein anderes Bearbeitungswerk 46 z. B. als ein Rillwerk ausgebildet. Das betreffende Bearbeitungswerk 46 ist die mechanische Weiterverarbeitung der Bogen vorzugsweise in einem Zusammenwirken mit einem den jeweiligen Bogen transportierenden Zylinder ausführend ausgebildet. Nach ihrer mechanischen Weiterverarbeitung werden die Bogen und/oder aus ihnen herausgetrennte Nutzen z. B. mittels eines Kettenförderers 21 zur Auslage 12 transportiert und dort gesammelt, vorzugsweise gestapelt.

Die Bogen werden vom Ausgang der Offset-Druckeinrichtung 04 zumindest bis zum Ausgang des Zwischentrockners 07 oder Trockners 09, vorzugsweise bis zum Anfang der mechanischen Weiterverarbeitungseinrichtung 11 jeweils mittels einer mehrteiligen, d. h. aus mehreren in Transportrichtung T der Bogen nacheinander angeordneten Baugruppen, insbesondere Transporteinheiten bestehenden Transporteinrichtung transportiert, wobei die Transporteinrichtung mehrere Transportzylinder aufweist. Bei Bedarf kann auch zwischen der Offset-Druckeinrichtung 04 und der Non-Impact-Druckeinrichtung 06 ein Zwischentrockner 07 oder ein Trockner 09 angeordnet sein.

In den Fig. 3 bis 8 sind weitere Maschinenanordnungen jeweils mit mehreren Bearbeitungsstationen 01; 02; 03; 04; 06; 07; 08; 09; 11; 12 beispielhaft und schematisch dargestellt, wobei die jeweiligen Bezugszeichen die zuvor erläuterten Bearbeitungsstationen 01; 02; 03; 04; 06; 07; 08; 09; 11; 12 und weitere ihrer jeweiligen Aggregate bezeichnen.

In der Fig. 3 ist eine Maschinenanordnung mit folgenden in Transportrichtung T des Bedruckstoffes hintereinander angeordneten Bearbeitungsstationen 01; 02; 03; 04; 06; 07; 08; 09; 11; 12 gezeigt: Bogenanleger 01; Primerauftrageinrichtung 02 oder Lackiereinrichtung 08; Zwischentrockner 07; Non-Impact-Druckeinrichtung 06; Zwischentrockner 07; Lackiereinrichtung 08; Trockner 09; Auslage 12.

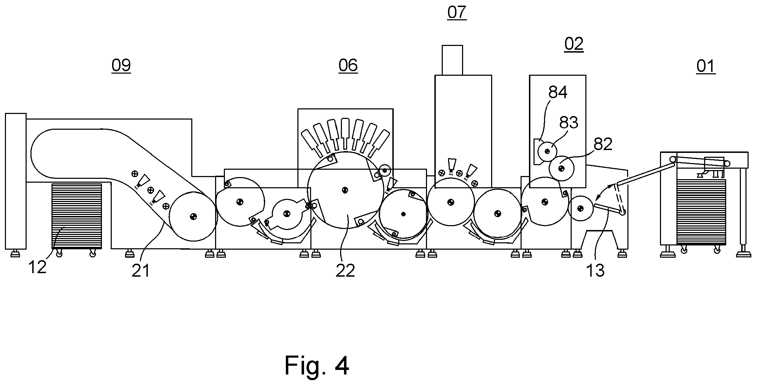

In der Fig. 4 ist eine Maschinenanordnung mit folgenden in Transportrichtung T des Bedruckstoffes hintereinander angeordneten Bearbeitungsstationen 01; 02; 03; 04; 06; 07; 08; 09; 11; 12 gezeigt: Bogenanleger 01; Primerauftrageinrichtung 02; Zwischentrockner 07; Non-Impact-Druckeinrichtung 06; Trockner 09; Auslage 12.

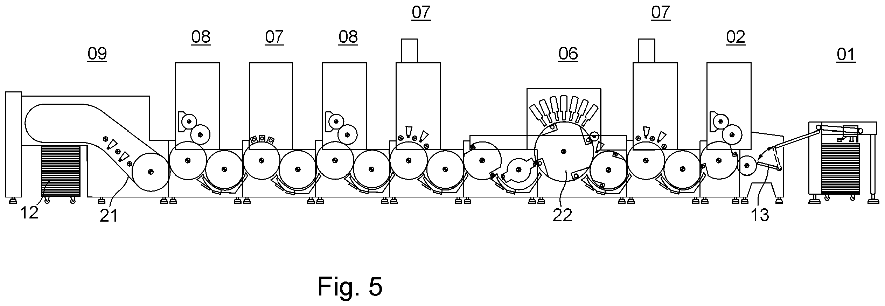

In der Fig. 5 ist eine Maschinenanordnung mit folgenden in Transportrichtung T des Bedruckstoffes hintereinander angeordneten Bearbeitungsstationen 01; 02; 03; 04; 06; 07; 08; 09; 11; 12 gezeigt: Bogenanleger 01; Primerauftrageinrichtung 02; Zwischentrockner 07; Non-Impact-Druckeinrichtung 06; Zwischentrockner 07; Lackiereinrichtung 08; Zwischentrockner 07; Lackiereinrichtung 08; Trockner 09; Auslage 12.

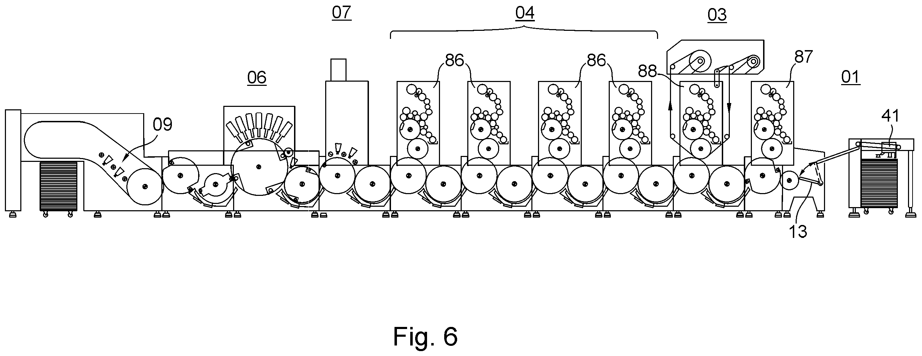

In der Fig. 6 ist eine Maschinenanordnung mit folgenden in Transportrichtung T des Bedruckstoffes hintereinander angeordneten Bearbeitungsstationen 01; 02; 03; 04; 06; 07; 08; 09; 11; 12 gezeigt: Bogenanleger 01; einer ersten Offset-Druckeinrichtung 04; Kaltfolienauftrageinrichtung 03; vier weiteren Offset-Druckeinrichtungen 04 in Reihenbauweise; Zwischentrockner 07; Non-Impact-Druckeinrichtung 06; Zwischentrockner 07; Non-Impact-Druckeinrichtung 06; Trockner 09; Auslage 12.

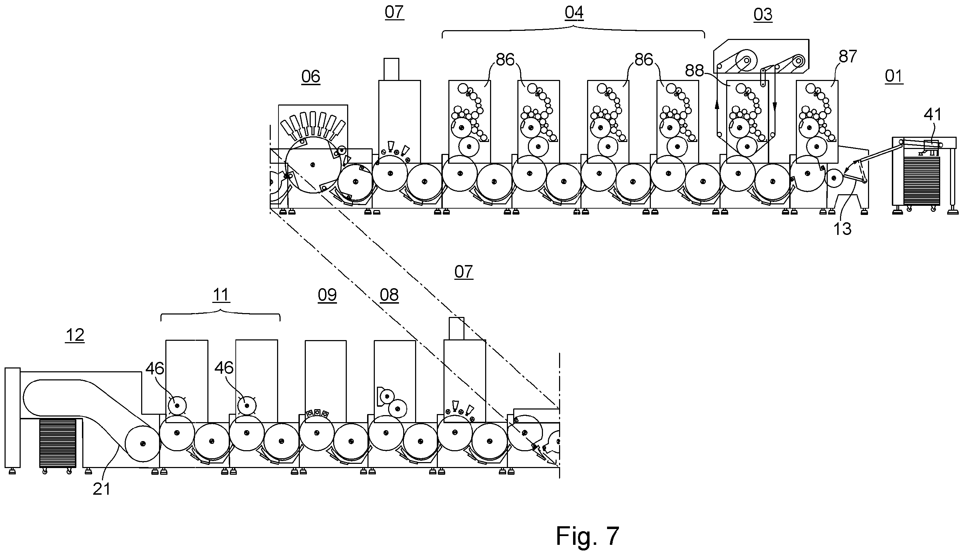

In der Fig. 7 ist eine aufgrund ihrer Länge in einem Versatz dargestellte Maschinenanordnung mit folgenden in Transportrichtung T des Bedruckstoffes hintereinander angeordneten Bearbeitungsstationen 01; 02; 03; 04; 06; 07; 08; 09; 11; 12 gezeigt: Bogenanleger 01; einer ersten Offset-Druckeinrichtung 04; Kaltfolienauftrageinrichtung 03; vier weiteren Offset-Druckeinrichtungen 04 in Reihenbauweise; Zwischentrockner 07; Non-Impact-Druckeinrichtung 06; Zwischentrockner 07; Lackiereinrichtung 08; Trockner 09; zwei mechanische Weiterverarbeitungseinrichtungen 11 in Reihenbauweise; Auslage 12.

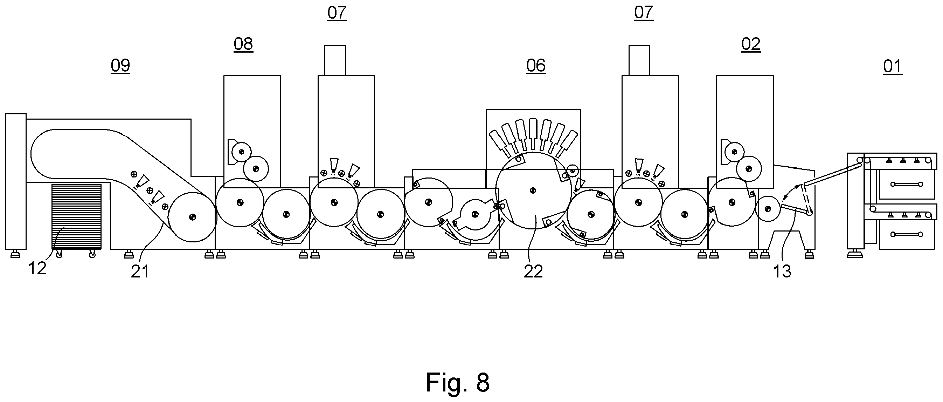

In der Fig. 8 ist eine Maschinenanordnung mit folgenden in Transportrichtung T des Bedruckstoffes hintereinander angeordneten Bearbeitungsstationen 01; 02; 03; 04; 06; 07; 08; 09; 11; 12 gezeigt: Magazinanleger 01; Primerauftrageinrichtung 02; Zwischentrockner 07; Non-Impact-Druckeinrichtung 06; Zwischentrockner 07; Lackiereinrichtung 08; Trockner 09; Auslage 12.

Wie bereits erwähnt, ist vorgesehen, mit den zuvor beschriebenen Maschinenanordnungen, die jeweils mehrere Bearbeitungsstationen 01; 02; 03; 04; 06; 07; 08; 09; 11; 12 zur Bearbeitung von Bogen und für den Transport dieser Bogen mindestens eine Transportvorrichtung aufweisen, Bogen unterschiedlichen Formats, d. h. von unterschiedlicher Länge und/oder Breite zu bearbeiten. Daher unterscheiden sich die i. d. R. rechteckförmigen Bogen z. B. in ihrer jeweiligen Länge, wobei sich diese Länge jeweils in Transportrichtung T dieser Bogen erstreckt. Um bei Verwendung einer insbesondere als eine Non-Impact-Druckeinrichtung 06 ausgebildeten Bearbeitungsstation 02; 03; 04; 06; 07; 08; 09; 11; 12, der Bogen sequentiell zugeführt werden, die Produktivität der jeweiligen Maschinenanordnung bei vergleichsweise kürzeren Bogen, d. h. bei Bogen kleineren Formats gegenüber sonst großformatigeren in dieser Maschinenanordnung bearbeiteten Bogen, nicht zu verringern, wird ein Verfahren mit folgenden Verfahrensschritten vorgeschlagen:
Verfahren zum Betrieb einer mehrere Bogen einer Bearbeitungsstation 02; 03; 04; 06; 07; 08; 09; 11; 12 sequentiell zuführenden Transportvorrichtung, bei dem zum Bearbeiten durch dieselbe Bearbeitungsstation 02; 03; 04; 06; 07; 08; 09; 11; 12 Bogen unterschiedlicher sich jeweils in Transportrichtung T dieser Bogen erstreckender Länge verwendet werden, wobei die der Bearbeitungsstation 02; 03; 04; 06; 07; 08; 09; 11; 12 nacheinander zuzuführenden Bogen von der Transportvorrichtung jeweils in einem Abstand transportiert werden, wobei die Transportvorrichtung den zu transportierenden Bogen jeweils eine Transportgeschwindigkeit aufprägt, wobei der zwischen unmittelbar aufeinander folgenden Bogen bestehende Abstand für Bogen verschiedener sich jeweils in Transportrichtung T dieser Bogen erstreckender Länge durch eine Veränderung der von der Transportvorrichtung den betreffenden Bogen aufzuprägenden Transportgeschwindigkeit konstant gehalten wird, wobei die Transportgeschwindigkeit des in Transportrichtung T nachfolgenden Bogens im Verhältnis zu der Transportgeschwindigkeit des unmittelbar vorausgehenden Bogens verändert wird. Dabei werden die der betreffenden Bearbeitungsstation 02; 03; 04; 06; 07; 08; 09; 11; 12 nacheinander zuzuführenden Bogen zum Erreichen und/oder zum Beibehalten einer von der Bearbeitungsstation 02; 03; 04; 06; 07; 08; 09; 11; 12 zu erbringenden hohen Produktivität von der Transportvorrichtung jeweils vorzugsweise in einem minimalen, aber i. d. R. von Null verschiedenen Abstand transportiert. Der Abstand zwischen in Transportrichtung T aufeinanderfolgenden Bogen, d. h. zwischen der sich quer zur Transportrichtung T erstreckenden hinteren Kante des vorhergehenden Bogens und der sich quer zur Transportrichtung T erstreckenden Vorderkante des unmittelbar nachfolgenden Bogens, liegt z. B. im Bereich zwischen 0,5 mm und 50 mm, vorzugsweise bei weniger als 10 mm. Wenn ein Bogen kürzerer Länge in der betreffenden Bearbeitungsstation 02; 03; 04; 06; 07; 08; 09; 11; 12 nach einem Bogen größerer Länge zu bearbeiten ist, wird der Bogen kürzerer Länge von der Transportvorrichtung durch eine Erhöhung seiner Transportgeschwindigkeit beschleunigt. Umgekehrt wird ein Bogen größerer Länge von der Transportvorrichtung durch eine Verringerung seiner Transportgeschwindigkeit verlangsamt, wenn der Bogen größerer Länge in der betreffenden Bearbeitungsstation 02; 03; 04; 06; 07; 08; 09; 11; 12 nach einem Bogen kürzerer Länge zu bearbeiten ist. Als Bearbeitungsstation 02; 03; 04; 06; 07; 08; 09; 11; 12 wird vorzugsweise eine Non-Impact-Druckeinrichtung 06 verwendet, deren Produktivität i. d. R. dann am größten ist, wenn ihr die von ihr zu bedruckenden Bogen ungeachtet ihres jeweiligen Formats in einem konstanten minimalen Abstand aufeinanderfolgend zugeführt werden. Wenn in der betreffenden Maschinenanordnung der Non-Impact-Druckeinrichtung 06 eine z. B. als eine Offset-Druckeinrichtung 04 ausgebildete Bearbeitungsstation 04 vorgeordnet ist, werden in der Offset-Druckeinrichtung 04 bedruckte Bogen unabhängig von ihrem jeweiligen Format mit der einer Produktionsgeschwindigkeit dieser Offset-Druckeinrichtung 04 entsprechenden Transportgeschwindigkeit der Transportvorrichtung zugeführt, wobei diese diesen Bogen von der Offset-Druckeinrichtung 04 vorgegebene Transportgeschwindigkeit während ihres Transports mit der Transportvorrichtung an die einer Verarbeitungsgeschwindigkeit der Non-Impact-Druckeinrichtung 06 entsprechende Transportgeschwindigkeit anzupassen ist. Wenn diese Bogen ungeachtet ihres jeweiligen Formats zusätzlich noch jeweils in einem konstanten Abstand zueinander der Non-Impact-Druckeinrichtung 06 zugeführt werden sollen, werden Bogen größerer Länge weniger verlangsamt als kürzere Bogen, jedoch wird in jedem Fall eine Verringerung ihrer jeweiligen Transportgeschwindigkeit erforderlich sein, weil die Verarbeitungsgeschwindigkeit der Non-Impact-Druckeinrichtung 06 i. d. R. geringer ist als die Produktionsgeschwindigkeit der Offset-Druckeinrichtung 04.

Der jeweilige Bogen wird während seines Transports von der Transportvorrichtung vorzugsweise jeweils kraftschlüssig z. B. durch Saugluft und/oder durch Greifer gehalten. In der bevorzugten Ausführung wird die dem betreffenden Bogen aufzuprägende Transportgeschwindigkeit von einer vorzugsweise elektronischen Steuereinheit eingestellt, wobei die Steuereinheit die Einstellung der Transportgeschwindigkeit insbesondere zur Einhaltung des konstanten Abstands zwischen aufeinanderfolgenden Bogen z. B. in einem Regelkreis vornimmt. Es ist z. B. vorgesehen, dass ein der mechanischen Weiterverarbeitungseinrichtung 11 zuzuführender Bogen durch den Schwinggreifer 19 und die Übergabetrommel 31 von der zweiten Transportgeschwindigkeit auf die dritte Transportgeschwindigkeit gebracht wird, was bedeutet, dass der betreffende Bogen insbesondere durch die von der Steuereinheit gesteuerte Rotation der Übergabetrommel 31 beschleunigt wird.

Fig. 9 zeigt eine weitere nicht erfindungsgemäße Maschinenanordnung mit mehreren i. d. R. verschiedenen Bearbeitungsstationen zum sequentiellen Bearbeiten mehrerer bogenförmiger Substrate. Die flächigen Substrate, die jeweils eine Vorderseite und eine Rückseite aufweisen, werden in einem Anleger 01 z. B. von einem Saugkopf 41 ergriffen und einzeln mittels eines Schwinggreifers 13 an eine Übergabetrommel 14 und von dort an einen rotierenden Anlagedruckzylinder 119 übergeben, wobei dieser Anlagedruckzylinder 119 auf seiner Mantelfläche jeweils mindestens eines dieser Substrate oder auch mehrere, z. B. zwei oder drei jeweils in Umfangsrichtung hintereinander angeordnete Substrate aufnimmt. Jedes der zu transportierenden Substrate ist an der Mantelfläche des Anlagedruckzylinder 119 mittels mindestens eines z. B. als Greifer ausgebildeten Halteelementes gehalten. Insbesondere biegeschlaffe und/oder dünne Substrate mit einer Dicke von z. B. bis zu 0,1 mm oder maximal 0,2 mm können z. B. auch durch Saugluft an der Mantelfläche des Anlagedruckzylinder 119 gehalten sein, wobei ein Aufliegen eines solchen Substrates auf der Mantelfläche des Anlagedruckzylinder 119, insbesondere an den Kanten dieses Substrates, z. B. durch insbesondere radial auf die Mantelfläche des Anlagedruckzylinder 119 gerichtete Blasluft unterstützt ist. An den Anlagedruckzylinder 119 ist in dessen Drehrichtung, die in der Fig. 9 durch einen Drehrichtungspfeil angedeutet ist, ausgehend von der an diesen Anlagedruckzylinder 119 angestellten Übergabetrommel 14 zunächst eine erste Primerauftrageinrichtung 02 zum Primern der Vorderseite und dieser ersten Primerauftrageinrichtung 02 nachfolgend eine zweite Primerauftrageinrichtung 126 zum Primern der Rückseite desselben bogenförmigen Substrates angestellt, wobei die zweite Primerauftrageinrichtung 126 die Rückseite des betreffenden Substrates z. B. indirekt primert, insbesondere durch eine Rückübertragung des von dieser zweiten Primerauftrageinrichtung 126 auf die Mantelfläche des Anlagedruckzylinders 119 aufgetragenen Primers von dieser Mantelfläche auf die Rückseite des betreffenden Substrates. Das Primern der Vorderseite und/oder Rückseite des betreffenden Substrates kann je nach Bedarf jeweils vollflächig oder teilflächig erfolgen. Der Anlagedruckzylinder 119 übergibt ein beidseitig geprimertes Substrat an eine erste Transportvorrichtung, z. B. eine mindestens ein Zugorgan aufweisende, insbesondere endlos umlaufende Transportvorrichtung, z. B. an einen ersten Kettenförderer 16, wobei diese erste Transportvorrichtung dieses Substrat zu einer ersten Non-Impact-Druckeinrichtung 06 transportiert, wobei diese erste Non-Impact-Druckeinrichtung 06 die Vorderseite des betreffenden Substrates zumindest teilweise bedruckt. Die erste Non-Impact-Druckeinrichtung 06 überträgt das vorderseitig bedruckte Substrat an eine zweite Transportvorrichtung, z. B. eine mindestens ein Zugorgan aufweisende, insbesondere endlos umlaufende Transportvorrichtung, z. B. an einen zweiten Kettenförderer 21, wobei diese zweite Transportvorrichtung das betreffende Substrat z. B. im Bereich seines ersten Kettenrades aufnimmt. Beispielsweise im Bereich des zweiten Kettenrades dieses zweiten Kettenförderers 21 ist eine zweite Non-Impact-Druckeinrichtung 127 angeordnet, wobei diese zweite Non-Impact-Druckeinrichtung 127 die Rückseite des betreffenden zuvor vorderseitig bedruckten Substrates zumindest teilweise bedruckt. Damit sind die erste Non-Impact-Druckeinrichtung 06 und die zweite Non-Impact-Druckeinrichtung 127 in Transportrichtung T des jeweiligen bogenförmigen Substrates an verschiedenen Positionen des Transportweges des betreffenden Substrates nacheinander angeordnet. Das betreffende nun beidseitig bedruckte Substrat wird anschließend z. B. auf einem Stapel in einer Auslage 12 abgelegt.

Die in der Fig. 9 dargestellte das betreffende Substrat beidseitig bearbeitende Maschinenanordnung weist jeweils mehrere, vorzugsweise vier Trockner 121; 122; 123; 124 auf, und zwar einen ersten Trockner 121 zum Trocknen des auf der Vorderseite des betreffenden Substrates aufgetragenen Primers und einen zweiten Trockner 122 zum Trocknen des auf der Rückseite des betreffenden Substrates aufgetragenen Primers. Überdies sind ein dritter Trockner 123 zum Trocknen des betreffenden mit der ersten Non-Impact-Druckeinrichtung 06 vorderseitig bedruckten Substrates und ein vierter Trockner 124 zum Trocknen des betreffenden mit der zweiten Non-Impact-Druckeinrichtung 127 rückseitig bedruckten Substrates vorgesehen. Die z. B. baugleich ausgebildeten Trockner 121; 122; 123; 124 sind das betreffende Substrat z. B. durch eine Bestrahlung mit infraroter oder ultravioletter Strahlung trocknend ausgebildet, wobei die Strahlungsart insbesondere davon abhängig ist, ob die auf das betreffende Substrat aufgetragene Druckfarbe oder Tinte wasserbasiert oder UV-härtend ist. Die Transportrichtung T des betreffenden durch die Maschinenanordnung transportierten Substrates ist in der Fig. 9 jeweils durch Pfeile angedeutet. Die erste Non-Impact-Druckeinrichtung 06 und die zweite Non-Impact-Druckeinrichtung 127 sind jeweils z. B. als mindestens eine Inkjetdruckeinrichtung ausgebildet. Im Wirkungsbereich der ersten Non-Impact-Druckeinrichtung 06 ist eine dritte Transportvorrichtung angeordnet, die das betreffende beidseitig geprimerte Substrat von der ersten Transportvorrichtung übernimmt, zur zweiten Transportvorrichtung transportiert und an diese zweite Transportvorrichtung abgibt. Die das betreffende Substrat im Wirkungsbereich der ersten Non-Impact-Druckeinrichtung 06 transportierende dritte Transportvorrichtung ist als ein Transportzylinder 128 ausgebildet, wobei die vorzugsweise mehreren Inkjetdruckeinrichtungen der ersten Non-Impact-Druckeinrichtung 06 jeweils radial zu diesem Transportzylinder 128 angeordnet sind.

Die das betreffende Substrat im Wirkungsbereich der ersten Non-Impact-Druckeinrichtung 06 transportierende dritte Transportvorrichtung und die das betreffende Substrat im Wirkungsbereich der zweiten Non-Impact-Druckeinrichtung 127 transportierende zweite Transportvorrichtung weisen jeweils vorzugsweise einen Einzelantrieb auf, wobei diese Einzelantriebe jeweils z. B. als ein in seiner jeweiligen Drehzahl und/oder Winkellage geregelter oder zumindest regelbarer vorzugsweise elektrisch angetriebener Motor ausgebildet sind, wobei mittels dieser die betreffenden Transportvorrichtungen in ihrem jeweiligen Bewegungsverhalten beeinflussenden Einzelantriebe das Bedrucken des betreffenden Substrates auf dessen Vorderseite durch die erste Non-Impact-Druckeinrichtung 06 und auf dessen Rückseite durch die zweite Non-Impact-Druckeinrichtung 127 synchronisiert oder zumindest synchronisierbar ist.

In einer bevorzugten Ausführung ist der erste Trockner 121 zum Trocknen des auf der Vorderseite des betreffenden Substrates aufgetragenen Primers z. B. im Bereich des Anlagedruckzylinders 119 angeordnet. Der zweite Trockner 122 zum Trocknen des auf der Rückseite des betreffenden Substrates aufgetragenen Primers ist vorzugsweise im Bereich der ersten Transportvorrichtung angeordnet. Der dritte Trockner 123 zum Trocknen des betreffenden mit der ersten Non-Impact-Druckeinrichtung 06 vorderseitig bedruckten Substrates ist z. B. im Bereich der zweiten Transportvorrichtung angeordnet oder befindet sich im Bereich der dritten Transportvorrichtung, welche sich ihrerseits im Wirkungsbereich der ersten Non-Impact-Druckeinrichtung 06 befindet und mit dieser zusammenwirkt. Der vierte Trockner 124 zum Trocknen des betreffenden mit der zweiten Non-Impact-Druckeinrichtung 127 rückseitig bedruckten Substrates ist in Transportrichtung T des betreffenden durch die Maschinenanordnung transportierten Substrates z. B. nach der dritten Transportvorrichtung angeordnet.

Die in der Fig. 9 dargestellte Maschinenanordnung ist auch als eine Maschinenanordnung zum sequentiellen Bearbeiten mehrerer bogenförmiger jeweils eine Vorderseite und eine Rückseite aufweisender Substrate beschreibbar, wobei eine erste Non-Impact-Druckeinrichtung 06 und eine zweite Non-Impact-Druckeinrichtung 127 sowie eine erste Primerauftrageinrichtung 02 und eine zweite Primerauftrageinrichtung 126 vorgesehen sind, wobei jeweils hinsichtlich desselben bogenförmigen Substrates die erste Primerauftrageinrichtung 02 die Vorderseite primernd und die zweite Primerauftrageinrichtung 126 die Rückseite primernd angeordnet sind und wobei hinsichtlich dieses Substrates die erste Non-Impact-Druckeinrichtung 06 die von der ersten Primerauftrageinrichtung 02 geprimerte Vorderseite bedruckend und die zweite Non-Impact-Druckeinrichtung 127 die von der zweiten Primerauftrageinrichtung 126 geprimerte Rückseite bedruckend angeordnet sind. Dabei sind ein erster Trockner 121 zum Trocknen des auf der Vorderseite des betreffenden Substrates aufgetragenen Primers in Transportrichtung T des betreffenden Substrates vor der ersten Non-Impact-Druckeinrichtung 06 und ein zweiter Trockner 122 zum Trocknen des auf der Rückseite des betreffenden Substrates aufgetragenen Primers in Transportrichtung T des betreffenden Substrates vor der zweiten Non-Impact-Druckeinrichtung 127 und ein dritter Trockner 123 zum Trocknen des betreffenden mit der ersten Non-Impact-Druckeinrichtung 06 vorderseitig bedruckten Substrates in Transportrichtung T des betreffenden Substrates nach der ersten Non-Impact-Druckeinrichtung 06 und ein vierter Trockner 124 zum Trocknen des betreffenden mit der zweiten Non-Impact-Druckeinrichtung 127 rückseitig bedruckten Substrates in Transportrichtung T des betreffenden Substrates nach der zweiten Non-Impact-Druckeinrichtung 127 vorgesehen.

Die zweite Primerauftrageinrichtung 126 kann dabei in Transportrichtung T des betreffenden Substrates wahlweise vor oder nach der zweiten Non-Impact-Druckeinrichtung 127 angeordnet sein. Der erste Trockner 121 zum Trocknen des auf der Vorderseite des betreffenden Substrates aufgetragenen Primers und/oder der zweite Trockner 122 zum Trocknen des auf der Rückseite des betreffenden Substrates aufgetragenen Primers und/oder der dritte Trockner 123 zum Trocknen des betreffenden mit der ersten Non-Impact-Druckeinrichtung 06 vorderseitig bedruckten Substrates und/oder der vierte Trockner 124 zum Trocknen des betreffenden mit der zweiten Non-Impact-Druckeinrichtung 127 rückseitig bedruckten Substrates sind jeweils z. B. als ein das betreffende geprimerte und/oder bedruckte Substrat durch Heißluft und/oder durch eine Bestrahlung mit infraroter oder ultravioletter Strahlung trocknender Trockner ausgebildet, wobei der das betreffende geprimerte und/oder bedruckte Substrat durch eine Bestrahlung mit infraroter oder ultravioletter Strahlung trocknende Trockner 121; 122; 123; 124 vorzugsweise als ein LED-Trockner ausgebildet ist, also als ein jeweils Halbleiterdioden verwendender Trockner. Zudem ist mindestens eine das betreffende Substrat transportierende Transportvorrichtung vorgesehen, wobei diese Transportvorrichtung als ein Transportzylinder oder als ein umlaufendes Transportband oder als ein Kettenförderer ausgebildet ist. Dabei weist die mindestens eine das betreffende Substrat transportierende Transportvorrichtung mindestens ein Halteelement auf, wobei das mindestens eine Halteelement das betreffende Substrat durch einen Kraftschluss oder durch einen Formschluss haltend ausgebildet ist.

Die Fig. 10 zeigt noch eine weitere vorteilhafte nicht erfindungsgemäße Maschinenanordnung zum sequentiellen Bearbeiten mehrerer bogenförmiger jeweils eine Vorderseite und eine Rückseite aufweisender Substrate. Diese vorzugsweise als eine Druckmaschine, insbesondere als eine Bogendruckmaschine ausgebildete Maschinenanordnung weist zumindest einen ersten Druckzylinder 117 und einen zweiten Druckzylinder 118 auf. Dabei sind jeweils am Umfang des ersten Druckzylinders 117 mindestens eine die Vorderseite des betreffenden Substrates bedruckende erste Non-Impact-Druckeinrichtung 06 und in Drehrichtung des ersten Druckzylinders 117 nach der ersten Non-Impact-Druckeinrichtung 06 ein die von der ersten Non-Impact-Druckeinrichtung 06 bedruckte Vorderseite des betreffenden Substrates trocknender Trockner 123 sowie jeweils am Umfang des zweiten Druckzylinders 118 mindestens eine die Rückseite des betreffenden Substrates bedruckende zweite Non-Impact-Druckeinrichtung 127 und in Drehrichtung des zweiten Druckzylinders 118 nach der zweiten Non-Impact-Druckeinrichtung 127 ein die von der zweiten Non-Impact-Druckeinrichtung 127 bedruckte Rückseite des betreffenden Substrates trocknender Trockner 124 angeordnet. Die erste Non-Impact-Druckeinrichtung 06 und die zweite Non-Impact-Druckeinrichtung 127 sind z. B. jeweils als mindestens eine Inkjetdruckeinrichtung ausgebildet. Beispielsweise verdrucken die erste Non-Impact-Druckeinrichtung 06 und/oder die zweite Non-Impact-Druckeinrichtung 127 jeweils mehrere, z. B. vier Druckfarben, insbesondere die Druckfarben Gelb, Magenta, Cyan und Schwarz, wobei für jede dieser Druckfarben bezüglich der betreffenden Non-Impact-Druckeinrichtung 06; 127 jeweils vorzugsweise eine bestimmte Inkjetdruckeinrichtung vorgesehen ist.

In der Maschinenanordnung gemäß der Fig. 10 sind der erste Druckzylinder 117 und der zweite Druckzylinder 118 einen gemeinsamen Walzenspalt bildend angeordnet, wobei der erste Druckzylinder 117 in diesem gemeinsamen Walzenspalt das betreffende vorderseitig bedruckte und getrocknete Substrat unmittelbar an den zweiten Druckzylinder 118 übergibt. In der bevorzugten Ausführung dieser Maschinenanordnung sind zudem eine erste Primerauftrageinrichtung 02 und eine zweite Primerauftrageinrichtung 126 vorgesehen, wobei jeweils hinsichtlich desselben bogenförmigen Substrates die erste Primerauftrageinrichtung 02 die Vorderseite primernd und die zweite Primerauftrageinrichtung 126 die Rückseite primernd angeordnet sind, wobei hinsichtlich dieses Substrates die erste Non-Impact-Druckeinrichtung 06 die von der ersten Primerauftrageinrichtung 02 geprimerte Vorderseite bedruckend und die zweite Non-Impact-Druckeinrichtung 127 die von der zweiten Primerauftrageinrichtung 126 geprimerte Rückseite bedruckend angeordnet sind. Die erste Primerauftrageinrichtung 02 und die zweite Primerauftrageinrichtung 126 weisen jeweils z. B. einen Anlagedruckzylinder 119 auf, wobei diese beiden Anlagedruckzylinder 119 einen gemeinsamen Walzenspalt bildend angeordnet sind, wobei der die erste Primerauftrageinrichtung 02 aufweisende Anlagedruckzylinder 119 in diesem gemeinsamen Walzenspalt das betreffende Substrat unmittelbar an den die zweite Primerauftrageinrichtung 126 aufweisenden Anlagedruckzylinder 119 übergibt. Dabei sind der die zweite Primerauftrageinrichtung 126 aufweisende Anlagedruckzylinder 119 und der die erste Non-Impact-Druckeinrichtung 06 aufweisende erste Druckzylinder 117 einen gemeinsamen Walzenspalt bildend angeordnet, wobei der die zweite Primerauftrageinrichtung 126 aufweisende Anlagedruckzylinder 119 das betreffende Substrat unmittelbar an den die erste Non-Impact-Druckeinrichtung 06 aufweisenden ersten Druckzylinder 117 übergibt.

Am Umfang des die erste Primerauftrageinrichtung 02 aufweisenden Anlagedruckzylinders 119 ist i. d. R. unmittelbar nach der ersten Primerauftrageinrichtung 02 z. B. ein die von dieser ersten Primerauftrageinrichtung 02 geprimerte Vorderseite des betreffenden Substrates trocknender Trockner 121 angeordnet und/oder am Umfang des die zweite Primerauftrageinrichtung 126 aufweisenden Anlagedruckzylinders 119 ist i. d. R. unmittelbar nach der zweiten Primerauftrageinrichtung 126 z. B. ein die von dieser zweiten Primerauftrageinrichtung 126 geprimerte Rückseite des betreffenden Substrates trocknender Trockner 122 angeordnet. Dabei ist bzw. sind der Trockner 121 zum Trocknen des auf der Vorderseite des betreffenden Substrates aufgetragenen Primers und/oder der Trockner 122 zum Trocknen des auf der Rückseite des betreffenden Substrates aufgetragenen Primers und/oder der Trockner 123 zum Trocknen des betreffenden mit der ersten Non-Impact-Druckeinrichtung 06 vorderseitig bedruckten Substrates und/oder der Trockner 124 zum Trocknen des betreffenden mit der zweiten Non-Impact-Druckeinrichtung 127 rückseitig bedruckten Substrates jeweils als ein das betreffende geprimerte und/oder bedruckte Substrat durch Heißluft und/oder durch eine Bestrahlung mit infraroter oder ultravioletter Strahlung trocknender Trockner ausgebildet. In einer besonders bevorzugten Ausführung ist der das betreffende geprimerte und/oder bedruckte Substrat durch eine Bestrahlung mit infraroter oder ultravioletter Strahlung trocknende Trockner 121; 122; 123; 124 als ein LED-Trockner ausgebildet, d. h. als ein die infrarote oder ultraviolette Strahlung jeweils mittels Halbleiterdioden erzeugender Trockner.

Überdies sind in der Maschinenanordnung gemäß der Fig. 10 der erste Druckzylinder 117 und der zweite Druckzylinder 118 und der die erste Primerauftrageinrichtung 02 aufweisende Anlagedruckzylinder 119 und der die zweite Primerauftrageinrichtung 126 aufweisende Anlagedruckzylinder 119 jeweils vorzugsweise in einem einzigen aus Zahnrädern gebildeten Antriebsstrang, d. h. in einem Zahnräderzug miteinander verbunden und in ihrer jeweiligen Rotation gemeinsam von einem einzigen Antrieb angetrieben, wobei dieser Antrieb vorzugsweise als ein insbesondere drehzahlgeregelter und/oder lagegeregelter Elektromotor ausgebildet ist. Der erste Druckzylinder 117 und der zweite Druckzylinder 118 und der die erste Primerauftrageinrichtung 02 aufweisende Anlagedruckzylinder 119 und der die zweite Primerauftrageinrichtung 126 aufweisende Anlagedruckzylinder 119 sind jeweils z. B. mehrfachgroß ausgebildet, d. h. auf deren Mantelfläche sind jeweils mehrere, z. B. zwei oder drei oder vier Substrate jeweils in Umfangsrichtung hintereinander angeordnet oder zumindest anordenbar. Jedes der zu transportierenden Substrate ist an der Mantelfläche des ersten Druckzylinders 117 und/oder des zweiten Druckzylinders 118 und/oder des die erste Primerauftrageinrichtung 02 aufweisenden Anlagedruckzylinders 119 und/oder des die zweite Primerauftrageinrichtung 126 aufweisenden Anlagedruckzylinder 119 jeweils mittels mindestens eines z. B. als Greifer ausgebildeten Halteelementes kraftschlüssig und/oder formschlüssig gehalten. Insbesondere biegeschlaffe und/oder dünne Substrate mit einer Dicke von z. B. bis zu 0,1 mm oder maximal 0,2 mm können kraftschlüssig z. B. durch Saugluft an der Mantelfläche des betreffenden Zylinders 117; 118; 119 gehalten sein, wobei ein Aufliegen eines solchen Substrates auf der Mantelfläche des betreffenden Zylinders 117; 118; 119, insbesondere an den Kanten dieses Substrates, z. B. durch insbesondere radial auf die Mantelfläche des betreffenden Zylinders 117; 118; 119 gerichtete Blasluft unterstützt ist.

Das betreffende beidseitig bedruckte Substrat wird im Anschluss nach seinem Transport durch den zweiten Druckzylinder 118 vorzugsweise mittels einer Transportvorrichtung z. B. zu einer Auslage 12 transportiert und dort in der Auslage 12 auf einem Stapel abgelegt. Die sich an den zweiten Druckzylinder 118 anschließende Transportvorrichtung ist z. B. als ein Kettenförderer ausgebildet, wobei das betreffende Substrat während seines Transports durch diese Transportvorrichtung vor seinem Ablegen in der Auslage 12 nochmals vorzugsweise beidseitig durch mindestens einen Trockner 09 getrocknet wird. In manchen Produktionslinien kann beabsichtigt sein, das betreffende von der ersten Non-Impact-Druckeinrichtung 06 vorderseitig und/oder von der zweiten Non-Impact-Druckeinrichtung 127 rückseitig bedruckte Substrat einseitig oder beidseitig mit weiteren Druckfarben, insbesondere Sonderfarben zu bedrucken und/oder z. B. durch einen Lackauftrag zu veredeln. In diesem letzteren Fall ist im Anschluss an den zweiten Druckzylinder 118 vor der das betreffende Substrat zu der Auslage 12 transportierenden Transportvorrichtung mindestens ein weiterer, z. B. ein dritter Druckzylinder oder vorzugsweise mindestens ein weiteres aus einem dritten Druckzylinder und einem vierten Druckzylinder gebildetes Zylinderpaar vorgesehen, an welchem mindestens einen weiteren z. B. dritten und/oder vierten Druckzylinder ähnlich wie am ersten Druckzylinder 117 und/oder am zweiten Druckzylinder 118 jeweils wieder eine weitere Druckeinrichtung, insbesondere eine weitere Non-Impact-Druckeinrichtung, oder mindestens eine Lackiereinrichtung 08 jeweils gegebenenfalls mit einem weiteren Trockner angeordnet. All diese aneinandergereihten Druckzylinder bilden in der betreffenden Maschinenanordnung dann einen durchgängigen Transportweg für das betreffende Substrat, wobei dieses Substrat dann jeweils von einem an den nächsten Druckzylinder übergeben wird. Das betreffende Substrat ist beidseitig bearbeitbar, insbesondere bedruckbar, ohne dass es in dieser Maschinenanordnung für dieses Substrat einer Wendeeinrichtung 23 bedarf. Die vorgeschlagene Maschinenanordnung ist damit sehr kompakt und kostengünstig aufgebaut. Die in der Fig. 10 dargestellte Maschinenanordnung ist besonders vorteilhaft i. V. m. UV-härtenden Druckfarben z. B. im Verpackungsdruck für Lebensmittel oder Kosmetika verwendbar.

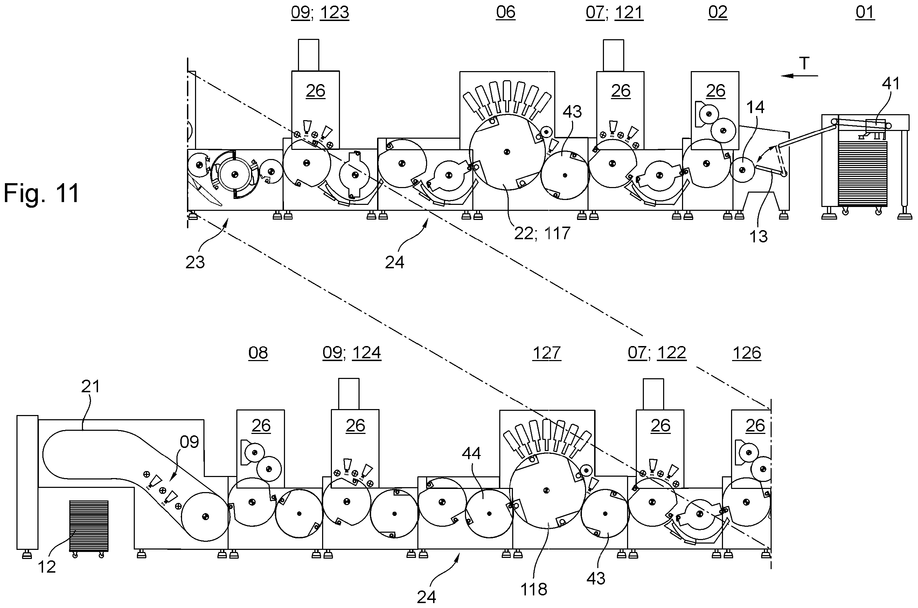

Fig. 11 zeigt beispielhaft eine Maschinenanordnung mit mehreren Bearbeitungsstationen 01; 02; 03; 04; 06; 07; 08; 09; 11; 12, wobei die Bearbeitungsstationen 01; 02; 03; 04; 06; 07; 08; 09; 11; 12 in Transportrichtung T der Substrate hintereinander angeordnet sind. Die Bearbeitungsstationen 01; 02; 03; 04; 06; 07; 08; 09; 11; 12 sind jeweils als ein eigenständig funktionsfähiges Modul ausgebildet, wobei jedes der Module jeweils eine in einem eigenen Gestell montierte Maschineneinheit bildet. In der bevorzugten Ausführung weisen diejenigen Module, die als eine Beschichtungseinrichtung 02; 03; 08 (d. h. Primerauftrageinrichtung 02; 126, Kaltfolienauftrageinrichtung 03 und Lackiereinrichtung 08) oder als ein Trockner 07; 09; 121; 122; 123; 124 oder als eine Druckeinrichtung 04; 06; 127 oder als eine mechanische Weiterverarbeitungseinrichtung 11 ausgebildet sind, jeweils eine Substratführungseinheit 24 und eine Substratbearbeitungseinheit 26 auf. Die Substratführungseinheit 24 weist zum Transport der Substrate z. B. eine Transportzylinderanordnung 17; 19 oder einen oder mehrere Transportzylinder 128 oder eine oder mehrere Transfertrommeln 43; 44 auf. Die Substratbearbeitungseinheit 26 weist je nach Art der jeweiligen Bearbeitungsstation 01; 02; 03; 04; 06; 07; 08; 09; 11; 12 z. B. die eigentliche Beschichtungseinrichtung 02; 03; 08 oder den Trockner 07; 09; 121; 122; 123; 124 oder mindestens ein Druckwerk 86; 87; 88 der Druckeinrichtung 04; 06; 127 oder mindestens ein Bearbeitungswerk 46 der mechanischen Weiterverarbeitungseinrichtung 11 auf.

Die in der Fig. 11 dargestellte Maschinenanordnung ist eine Maschinenanordnung für den Schön- und Widerdruck und weist ausgehend von einem als Bogenanleger 01 oder Magazinanleger 01 ausgebildeten, gestapelte Substrate nacheinander z. B. mit einem Saugkopf 41 ergreifenden Anleger 01 und einem nachgeordneten Schwinggreifer 13 mit einer Übergabetrommel 14 in Transportrichtung T der Substrate (Bogen) hintereinander eine Beschichtungseinrichtung 02; 03; 08 insbesondere in Form einer ersten Primerauftrageinrichtung 02 und danach einen ersten Trockner 07; 121 auf. Die derart vorderseitig vorbehandelten Substrate werden dann einer jeweils die Vorderseite der Substrate bedruckenden ersten Non-Impact-Druckeinrichtung 06 mit einem ersten Druckzylinder 22; 117 zugeführt, wobei dieser erste Druckzylinder 22; 117 vorzugsweise dreifachgroß oder vierfachgroß ausgebildet ist, was bedeutet, dass dieser erste Druckzylinder 22; 117 an seinem Umfang mindestens so viele Halteelemente aufweist, dass an seinem Umfang hintereinander drei oder vier Substrate jeweils durch einen Kraftschluss und/oder durch einen Formschluss haltend angeordnet oder zumindest anordenbar sind. Im Regelfall ist jedem am Umfang des Druckzylinders 22; 117 zu haltenden Substrat jeweils mindestens ein Halteelement zugeordnet, wobei diejenigen Halteelemente, die unterschiedlichen Substraten zugeordnet sind, jeweils unabhängig voneinander, d. h. getrennt voneinander betätigbar sind. Als Greifer ausgebildete Halteelemente sind insbesondere in einem Kanal angeordnet, wobei sich der jeweilige Kanal jeweils an der Mantelfläche des betreffenden Druckzylinders 22; 117 axial erstreckt. Das bedeutet bei z. B. vier entlang des Umfangs des betreffenden Druckzylinders 22; 117 anordenbaren Substraten, dass der betreffende Druckzylinder 22; 117 vier Kanäle aufweist, wobei in jedem Kanal mindestens ein Halteelement angeordnet ist. In einem Kanal können z. B. auch mindestens zwei Haltelemente angeordnet sein, wobei eines dieser Halteelemente eine in Transportrichtung T der Substrate hintere Kante von einem ersten dieser Substrate hält und ein anderes dieser Halteelemente eine in Transportrichtung T der Substrate vordere Kante von einem dem ersten Substrat am Umfang des betreffenden Druckzylinders 22; 117 unmittelbar nachfolgenden zweiten Substrat hält. Entlang eines Teils des Umfangs des ersten Druckzylinders 22; 117 sind nacheinander vorzugsweise mehrere Inkjetdruckeinrichtungen angeordnet. In der bevorzugten Ausführung folgt der ersten Non-Impact-Druckeinrichtung 06 eine als ein reines Transportmodul ausgebildete Substratführungseinheit 24 ohne weitere Substratbearbeitungseinheit 26. Auch dieses Transportmodul ist in einem eigenen Gestell angeordnet. Diese Substratführungseinheit 24 ermöglicht in dieser Maschinenanordnung die Ausbildung einer ausreichend breiten Quergalerie, welche ihrerseits z. B. für Wartungs- und/oder Reparaturarbeiten die Zugänglichkeit zur ersten Non-Impact-Druckeinrichtung 06 verbessert. Nach der Substratführungseinheit 24 ist ein die bedruckte Vorderseite der Substrate trocknender zweiter Trockner 09; 123 angeordnet. Dem zweiten Trockner 09; 123 folgt eine Wendeeinrichtung 23, die es ermöglicht, dass im Fortgang auch die Rückseite der Substrate bedruckt werden kann. So wie zuvor für den Schöndruck, d. h. für das Bedrucken der Vorderseite beschrieben, werden die Substrate von der Wendeeinrichtung 23 kommend zuerst einer die Rückseite der Substrate behandelnden zweiten Primerauftrageinrichtung 126 und danach einem dritten Trockner 07; 122 zugeführt. Es folgt eine jeweils die Rückseite der Substrate bedruckende zweite Non-Impact-Druckeinrichtung 127 mit einem zweiten Druckzylinder 118, wobei dieser zweite Druckzylinder 118 wiederum vorzugsweise dreifachgroß oder vierfachgroß ausgebildet ist, was bedeutet, dass dieser zweite Druckzylinder 118 an seinem Umfang so viele Halteelemente aufweist, dass an seinem Umfang hintereinander drei oder vier Substrate jeweils durch einen Kraftschluss und/oder durch einen Formschluss haltend angeordnet oder zumindest anordenbar sind. Entlang eines Teils des Umfangs des zweiten Druckzylinders 118 sind nacheinander vorzugsweise mehrere Inkjetdruckeinrichtungen angeordnet. Auch nach der zweiten Non-Impact-Druckeinrichtung 127 ist aus demselben Grund wie zuvor erläutert vorzugsweise eine Substratführungseinheit 24 ohne weitere Substratbearbeitungseinheit 26 angeordnet. Es folgt ein vierter Trockner 09; 124 zum Trocknen der bedruckten Rückseite der Substrate. In der bevorzugten Ausführung ist danach eine Lackiereinrichtung 08 vorgesehen. Die lackierten Substrate werden anschließend in einem weiteren Trockner 09 getrocknet, wobei dieser Trockner 09 z. B. im Transportweg einer als ein Kettenförderer 21 ausgebildeten Transporteinrichtung angeordnet ist, wobei diese Transporteinrichtung die Substrate zu einer Auslage 12, insbesondere zu einer Mehrstapelauslage transportiert und dort auslegt. In der Maschinenanordnung der Fig. 11 sind die jeweiligen jeweils eine Transportzylinderanordnung 17; 19 aufweisenden Substratführungseinheiten 24 abgesehen von den beiden Druckzylindern 22; 117; 118 vorzugsweise jeweils doppeltgroß ausgebildet, so dass am Umfang der jeweiligen Transportzylinder 128 oder Transfertrommeln 43; 44 jeweils zwei Substrate hintereinander angeordnet oder zumindest anordenbar sind.

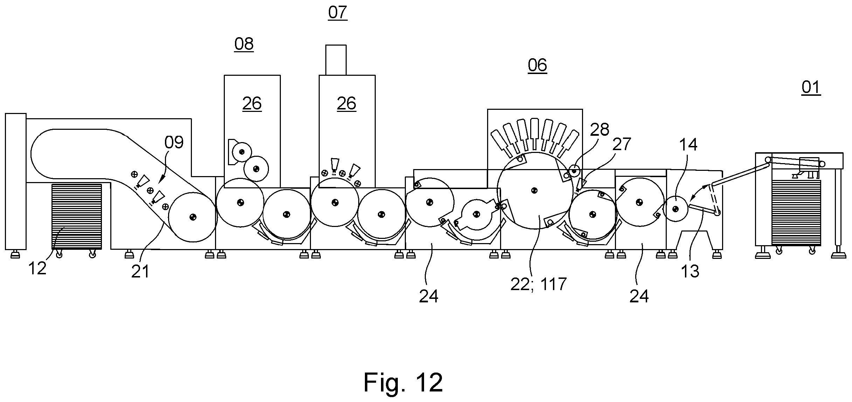

Fig. 12 zeigt beispielhaft eine Maschinenanordnung für die einseitige Bearbeitung von Substraten, insbesondere für deren einseitigen Druck. Die Substrate werden von einem Anleger 01 kommend mittels eines Schwinggreifers 13 einer Übergabetrommel 14 übergeben und von dort über eine z. B. nur einen einzigen Transportzylinder 128 oder nur eine einzige Transfertrommel 43; 44 aufweisenden Substratführungseinheit 24 einer jeweils die Vorderseite der Substrate bedruckenden Non-Impact-Druckeinrichtung 06 mit einem dreifachgroß oder vierfachgroß ausgebildeten Druckzylinder 22; 117 zugeführt. Um die Auflage der Substrate auf der Mantelfläche des Druckzylinders 22; 117 zu verbessern, sind z. B. eine Blaslufteinrichtung 27 und/oder ein Andrückelement 28 z. B. in Form einer Glättwalze vorgesehen. Es folgt eine Substratführungseinheit 24 mit einer mindestens zwei Transportzylinder 128 oder Transfertrommeln 43; 44 aufweisenden Transportzylinderanordnung 17; 19. Es schließen sich danach ein Trockner 07 und eine Lackiereinrichtung 08 an. Die lackierten Substrate werden anschließend in einem weiteren Trockner 09 getrocknet, wobei dieser Trockner 09 z. B. wieder im Transportweg einer als ein Kettenförderer 21 ausgebildeten Transporteinrichtung angeordnet ist, wobei diese Transporteinrichtung die Substrate zu einer Auslage 12 transportiert und dort auslegt. Die Substratführungseinheiten 24 weisen mit Ausnahme des Druckzylinders 22; 117 vorzugsweise jeweils doppeltgroß ausgebildete Transportzylinder 128 oder Transfertrommeln 43; 44 auf. Eine Substratführungseinheit 24 mit einer mindestens zwei Transportzylinder 128 oder Transfertrommeln 43; 44 aufweisenden Transportzylinderanordnung 17; 19 erstreckt sich in Transportrichtung T der Substrate über eine Länge, die mindestens dem eineinhalbfachen Durchmesser des betreffenden Transportzylinders 128 oder der betreffenden Transfertrommel 43; 44 entspricht.

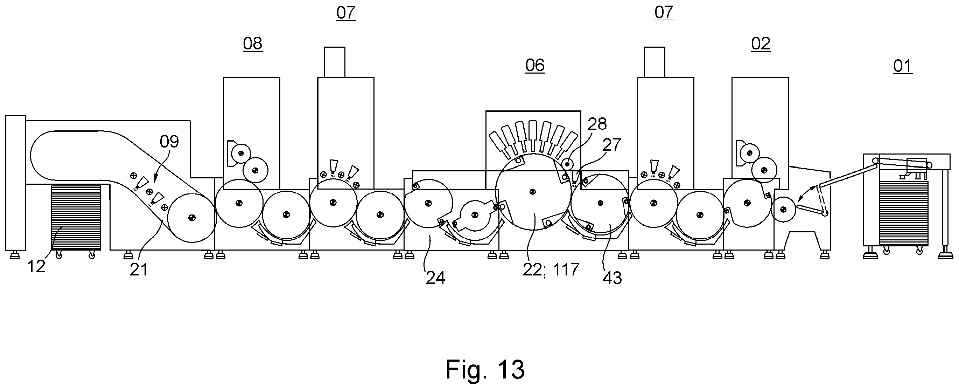

Die Figuren 13 bis 15 zeigen beispielhaft jeweils eine Maschinenanordnung für die einseitige Bearbeitung von Substraten, insbesondere für deren einseitigen Druck, wobei nach dem Anleger 01 z. B. eine Primerauftrageinrichtung 02 und ein Trockner 07 vorgesehen sind. Es folgen in der Maschinenanordnung eine Non-Impact-Druckeinrichtung 06, eine Substratführungseinheit 24, ein weiterer Trockner 07, eine Lackiereinrichtung 08 und ein z. B. im Transportweg einer als ein Kettenförderer 21 ausgebildeten Transporteinrichtung angeordneter Trockner 09, wobei diese Transporteinrichtung die Substrate zu einer Auslage 12 transportiert und dort auslegt.

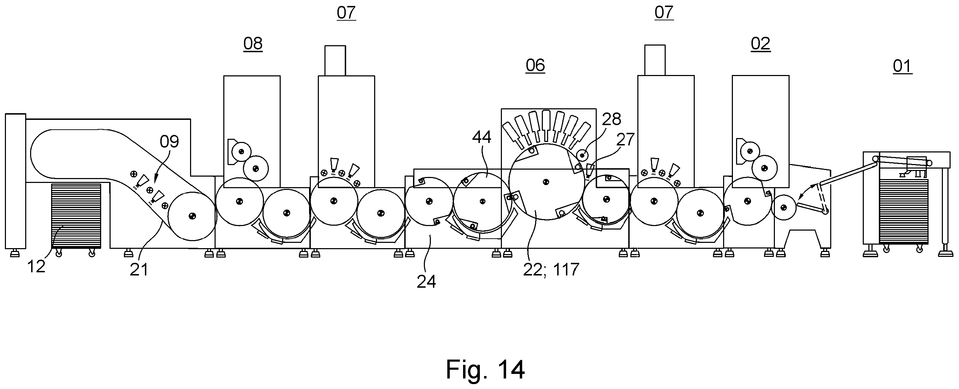

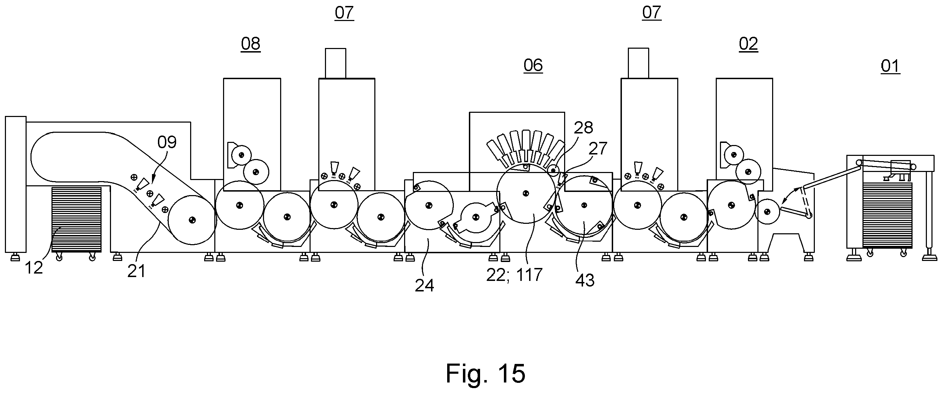

In der Maschinenanordnung der Fig. 13 ist der Druckzylinder 22; 117 vierfachgroß ausgebildet. Der vierfachgroße Druckzylinder 22; 117 übernimmt die zu bedruckenden Substrate von einer unmittelbar vorgeordneten Transfertrommel 43, die dreifachgroß ausgebildet ist. In der Maschinenanordnung der Fig. 14 ist der Druckzylinder 22; 117 ebenfalls vierfachgroß ausgebildet, jedoch übergibt der vierfachgroße Druckzylinder 22; 117 die bedruckten Substrate an eine diesem Druckzylinder 22; 117 unmittelbar nachgeordnete dreifachgroß ausgebildete Transfertrommel 44. Fig. 14 zeigt den vierfachgroß ausgebildeten Druckzylinders 22; 117 mit einer diesem Druckzylinder 22; 117 unmittelbar vorgeordneten doppeltgroß ausgebildeten Transfertrommel 43. Die Mantelfläche des Druckzylinders 22; 117 und die Transfertrommel 43 sind durch Ausbildung eines gemeinsamen Walzenstreifens 32 vorzugsweise in einem Berührungskontakt zueinander angeordnet. In der nicht erfindungsgemäßen Maschinenanordnung der Fig. 15 sind der Druckzylinder 22; 117 und die diesem Druckzylinder 22; 117 unmittelbar vorgeordnete Transfertrommel 43 jeweils dreifachgroß ausgebildet. Die Maschinenanordnungen der Figuren 13 bis 15 unterscheiden sich jeweils durch das Format des Druckzylinders 22; 117 und einer diesem Druckzylinder 22; 117 unmittelbar vorgeordneten oder nachgeordneten Transfertrommel 43; 44. Ein vierfachgroß ausgebildeter Druckzylinder 22; 117 hat einen Durchmesser z. B. von etwa 1.200 mm. Eine doppeltgroß ausgebildete Transfertrommel 43 hat einen Durchmesser z. B. von etwa 600 mm. Das jeweilige Format von Druckzylinder 22; 117 und einer diesem Druckzylinder 22; 117 unmittelbar vorgeordneten oder nachgeordneten Transfertrommel 43; 44 ist durch die Anzahl der an deren jeweiligem Umfang angeordneten Halteelemente bestimmt, wobei die Halteelemente die jeweiligen Substrate jeweils durch einen Kraftschluss und/oder durch einen Formschluss am Umfang des betreffenden Druckzylinders 22; 117 oder der betreffenden Transfertrommel 43; 44 halten. Am Umfang des betreffenden Druckzylinders 22; 117 sind jeweils mehrere Inkjetdruckeinrichtungen und z. B. jeweils eine Blaslufteinrichtung 27 und/oder ein Andrückelement 28 z. B. in Form einer Glättwalze angeordnet.

### Bezugszeichenliste

- 01: Bearbeitungsstation; Anleger; Bogenanleger; Magazinanleger
- 02: Bearbeitungsstation; Primerauftrageinrichtung
- 03: Bearbeitungsstation; Kaltfolienauftrageinrichtung
- 04: Bearbeitungsstation; Offset-Druckeinrichtung; Flexo-Druckeinrichtung
- 05: -
- 06: Bearbeitungsstation; Non-Impact-Druckeinrichtung
- 07: Bearbeitungsstation; Zwischentrockner; Trockner
- 08: Bearbeitungsstation; Lackiereinrichtung
- 09: Bearbeitungsstation; Trockner
- 10: -
- 11: Bearbeitungsstation; mechanische Weiterverarbeitungseinrichtung
- 12: Bearbeitungsstation; Auslage
- 13: Schwinggreifer
- 14: Übergabetrommel
- 15: -
- 16: Greifersystem; Kettenförderer
- 17: Transportzylinderanordnung
- 18 19: Transportzylinderanordnung
- 20: -
- 21: Kettenförderer
- 22: Druckzylinder
- 23: Wendeeinrichtung
- 24: Substratführungseinheit
- 25: -
- 26: Substratbearbeitungseinheit
- 27: Blaslufteinrichtung
- 28: Andrückelement
- 29 bis 40 - 41: Saugkopf
- 42 - 43: Transfertrommel
- 44: Transfertrommel
- 45 - 46: Bearbeitungswerk
- 47 bis 81 - 82: Druckwerkszylinder 82
- 83: Auftragswalze; Rasterwalze
- 84: Rakel; Kammerrakelsystem
- 85: -
- 86: Druckwerk
- 87: Druckwerk
- 88: Druckwerk
- 89 bis 116 - 117: Druckzylinder 117
- 118: Druckzylinder
- 119: Anlagedruckzylinder
- 120 - 121: Trockner
- 122: Trockner
- 123: Trockner
- 124: Trockner
- 125: -
- 126: Primerauftrageinrichtung
- 127: Non-Impact-Druckeinrichtung
- 128: Transportzylinder
- T: Transportrichtung

## Patentansprüche

1. Maschinenanordnung zum sequentiellen Bearbeiten bogenförmiger Substrate, mit mehreren verschiedenen Bearbeitungsstationen, wobei zwei der entlang des Transportweges der Substrate angeordnete Bearbeitungsstationen jeweils eine die Substrate jeweils bedruckende jeweils als mindestens eine Inkjetdruckeinrichtung ausgebildete Non-Impact-Druckeinrichtung (06; 127) aufweisen, wobei die die Non-Impact-Druckeinrichtungen (06; 127) aufweisenden Bearbeitungsstationen jeweils einen Druckzylinder (22; 117; 118) aufweisen, wobei der in Transportrichtung (T) der Substrate erste Druckzylinder (22; 117) dreifachgroß oder vierfachgroß ausgebildet ist, wobei entlang eines Teils des Umfangs des ersten Druckzylinders (22; 117) nacheinander mehrere Inkjetdruckeinrichtungen angeordnet sind, wobei der in Transportrichtung (T) der Substrate zweite Druckzylinder (118) dreifachgroß oder vierfachgroß ausgebildet ist, wobei entlang eines Teils des Umfangs des zweiten Druckzylinders (22; 118) nacheinander mehrere Inkjetdruckeinrichtungen angeordnet sind, wobei zur Vorbehandlung der Vorderseite der Substrate eine erste Primereinrichtung (02) und ein dieser Primereinrichtung (02) nachgeordneter Trockner (07; 121) der den ersten Druckzylinder (22; 117) aufweisenden, die Vorderseite der Substrate bedruckenden Bearbeitungsstation vorgeordnet sind, **dadurch gekennzeichnet, dass** zwischen dem ersten Druckzylinder (22; 117) und dem zweiten Druckzylinder (118) eine Wendeeinrichtung (23) angeordnet ist, wobei in Transportrichtung (T) der Substrate nach der Wendeeinrichtung (23) und vor der den zweiten Druckzylinder (118) aufweisenden, die Rückseite der Substrate bedruckenden Bearbeitungsstation zuerst eine die Rückseite der Substrate behandelnde zweite Primerauftrageinrichtung (126) und danach ein dritter Trockner (07; 122) angeordnet sind, wobei auf der Mantelfläche der jeweiligen Druckzylinder (22; 117; 118) jeweils mehrere Substrate jeweils in Umfangsrichtung hintereinander angeordnet oder zumindest anordenbar sind, wobei der jeweilige Druckzylinder (22; 117; 118) an seinem Umfang jeweils mindestens so viele Halteelemente aufweist, dass an seinem Umfang hintereinander drei oder vier Substrate jeweils durch einen Kraftschluss und/oder durch einen Formschluss haltend angeordnet oder zumindest anordenbar sind.

2. Maschinenanordnung nach Anspruch 1, **dadurch gekennzeichnet, dass** in Transportrichtung (T) der Substrate nach der den ersten Druckzylinder (22; 117) aufweisenden Bearbeitungsstation und vor der Wendeeinrichtung (23) ein die bedruckte Vorderseite der Substrate trocknender zweiter Trockner (09; 123) angeordnet ist.

3. Maschinenanordnung nach Anspruch 1 oder 2, **dadurch gekennzeichnet, dass** in Transportrichtung (T) der betreffenden Substrate nach der den zweiten Druckzylinder (118) aufweisenden Bearbeitungsstation ein vierter Trockner (09; 124) zum Trocknen der bedruckten Rückseite der Substrate angeordnet ist.

4. Maschinenanordnung nach Anspruch 1 oder 2 oder 3, **dadurch gekennzeichnet, dass** in Transportrichtung (T) der betreffenden Substrate nach der den zweiten Druckzylinder (118) aufweisenden Bearbeitungsstation und/oder nach dem dieser Bearbeitungsstation nachgeordneten vierten Trockner (09; 124) eine Lackiereinrichtung (08) angeordnet ist.

5. Maschinenanordnung nach Anspruch 4, **dadurch gekennzeichnet, dass** der Lackiereinrichtung (08) ein weiterer Trockner (09) zum Trocknen der lackierten Substrate nachgeordnet ist.

6. Maschinenanordnung nach Anspruch 1 oder 2 oder 3 oder 4 oder 5, **dadurch gekennzeichnet, dass** diejenigen Halteelemente, die unterschiedlichen Substraten zugeordnet sind, jeweils unabhängig und getrennt voneinander betätigbar sind.

7. Maschinenanordnung nach Anspruch 1 oder 2 oder 3 oder 4 oder 5 oder 6, **dadurch gekennzeichnet, dass** die jeweils als eine Primerauftrageinrichtung (02; 126) oder als eine Lackiereinrichtung (08) oder als ein Trockner (07; 09; 121; 122; 123; 124) oder als eine Druckeinrichtung (06; 127) ausgebildeten Bearbeitungsstationen jeweils als ein eigenständig funktionsfähiges Modul ausgebildet sind, wobei jedes dieser Module jeweils eine in einem eigenen Gestell montierte Maschineneinheit bildet.

8. Maschinenanordnung nach Anspruch 7, **dadurch gekennzeichnet, dass** die Module jeweils eine Substratführungseinheit (24) und eine Substratbearbeitungseinheit (26) aufweisen, wobei die Substratführungseinheit (24) zum Transport der Substrate jeweils eine Transportzylinderanordnung (17; 19) oder einen oder mehrere Transportzylinder (128) oder eine oder mehrere Transfertrommeln (43; 44) aufweist.

9. Maschinenanordnung nach Anspruch 1 oder 2 oder 3 oder 4 oder 5 oder 6 oder 7 oder 8, **dadurch gekennzeichnet, dass** unmittelbar nach der den ersten Druckzylinder (22; 117) aufweisenden Bearbeitungsstation und/oder unmittelbar nach der den zweiten Druckzylinder (118) aufweisenden Bearbeitungsstation jeweils eine als ein reines Transportmodul ausgebildete Substratführungseinheit (24) ohne weitere Substratbearbeitungseinheit (26) angeordnet ist bzw. sind, wobei jedes dieser Transportmodule jeweils in einem eigenen Gestell angeordnet ist.

10. Maschinenanordnung nach Anspruch 8 oder 9, **dadurch gekennzeichnet, dass** die jeweiligen jeweils eine Transportzylinderanordnung (17; 19) aufweisenden Substratführungseinheiten (24) abgesehen von den beiden Druckzylindern (22; 117; 118) jeweils doppeltgroß ausgebildet sind, so dass am Umfang der jeweiligen Transportzylinder (128) oder Transfertrommeln (43; 44) jeweils zwei Substrate hintereinander angeordnet oder zumindest anordenbar sind.

11. Maschinenanordnung nach Anspruch 1 oder 2 oder 3 oder 4 oder 5 oder 6 oder 7 oder 8 oder 9 oder 10, **dadurch gekennzeichnet, dass** die Non-Impact-Druckeinrichtungen (06; 127) jeweils mehrere verschiedene Druckfarben verdrucken, wobei für jede dieser Druckfarben bezüglich der betreffenden Non-Impact-Druckeinrichtung (06; 127) jeweils eine bestimmte Inkjetdruckeinrichtung vorgesehen ist und/oder dass in Transportrichtung (T) der Substrate hintereinander vier oder fünf oder sechs oder sieben Non-Impact-Druckeinrichtungen (06; 127) angeordnet sind.

12. Maschinenanordnung nach Anspruch 1 oder 2 oder 3 oder 4 oder 5 oder 6 oder 7 oder 8 oder 9 oder 10 oder 11, **dadurch gekennzeichnet, dass** der Trockner (07; 121) zum Trocknen des jeweils auf die Vorderseite der betreffenden Substrate aufgetragenen Primers und/oder der Trockner (122) zum Trocknen des jeweils auf die Rückseite der betreffenden Substrate aufgetragenen Primers und/oder der Trockner (123) zum Trocknen der betreffenden mit der Non-Impact-Druckeinrichtung (06) jeweils vorderseitig bedruckten Substrate und/oder der Trockner (124) zum Trocknen der betreffenden mit der Non-Impact-Druckeinrichtung (127) jeweils rückseitig bedruckten Substrate und/oder der weitere Trockner (09) als ein die betreffenden geprimerten und/oder bedruckten und/oder lackierten Substrate jeweils durch Heißluft und/oder durch eine Bestrahlung mit infraroter oder ultravioletter Strahlung trocknender Trockner ausgebildet ist bzw. sind.

13. Maschinenanordnung nach Anspruch 1 oder 2 oder 3 oder 4 oder 5 oder 6 oder 7 oder 8 oder 9 oder 10 oder 11 oder 12, **dadurch gekennzeichnet, dass** die Halteelemente jeweils als Greifer ausgebildet sind.

14. Maschinenanordnung nach Anspruch 2 oder 3 oder 4 oder 5 oder 6 oder 7 oder 8 oder 9 oder 10 oder 11 oder 12 oder 13, **dadurch gekennzeichnet, dass** der zweite Trockner (09; 123) und/oder der vierte Trockner (09; 124) jeweils als ein Zwischentrockner ausgebildet sind.

15. Maschinenanordnung nach Anspruch 1 oder 2 oder 3 oder 4 oder 5 oder 6 oder 7 oder 8 oder 9 oder 10 oder 11 oder 12 oder 13 oder 14, **dadurch gekennzeichnet, dass** mindestens einer der Trockner (07; 09; 121; 122; 123; 124) als ein LED-Trockner ausgebildet ist.

16. Maschinenanordnung nach Anspruch 1 oder 2 oder 3 oder 4 oder 5 oder 6 oder 7 oder 8 oder 9 oder 10 oder 11 oder 12 oder 13 oder 14 oder 15, **dadurch gekennzeichnet, dass** ein Aufliegen der Substrate auf der Mantelfläche des jeweiligen Druckzylinders (22; 117; 118) durch auf die betreffende Mantelfläche gerichtete Blasluft unterstützt ist.

## Claims

1. A machine arrangement for sequential processing of sheet-type substrates having several different processing stations, wherein two processing stations arranged along the transport path of the substrates in each case have a non-impact printing device (06; 127) configured as at least one inkjet printing device respectively printing the substrates, wherein the processing stations having the non-impact printing devices (06; 127) each have a printing cylinder (22; 117; 118), wherein the first printing cylinder (22; 117) in the transport direction (T) of the substrates is configured triple sized or quadruple sized, wherein a plurality of inkjet printing devices are arranged one after the other along a portion of the circumference of the first printing cylinder (22; 117), wherein the second printing cylinder (118) in the transport direction (T) of the substrates is configured triple sized or quadruple sized, wherein a plurality of inkjet printing devices are arranged one after the other along a portion of the circumference of the second printing cylinder (22; 118), wherein for the preprocessing of the front side of the substrates a first primer device (02) and a dryer (07; 121) downstream of this primer device (02) having the first printing cylinder (22; 117) are arranged upstream of the processing station printing the front side of the substrates, **characterized in that** a turning device (23) is arranged between the first printing cylinder (22; 117) and the second printing cylinder (118), wherein in transport direction (T) of the substrates after the turning device (23) and before the processing station having the second printing cylinder (118) and printing the back side of the substrates first a second primer application device (126) processing the back side of the substrates and after that a third dryer (07; 122) are arranged, wherein several substrates are in each case arranged or at least can be arranged in circumferential direction in tandem on the lateral surface of the respective printing cylinders (22; 117; 118), wherein the respective printing cylinder (22; 117; 118) has at least as many retaining elements at its circumference that three or four substrates are arranged or at least can be arranged in tandem at its circumference in each case being held by a non-positive connection and/or by a positive connection.

2. The machine arrangement according to claim 1, **characterized in that** a second dryer (09; 123) drying the printed front side of the substrates is arranged in transport direction (T) of the substrates after the processing station having the first printing cylinder (22; 117) and before the turning device (23) .

3. The machine arrangement according to claim 1 or 2, **characterized in that** a fourth dryer (09; 124) for drying the printed back side of the substrates is arranged in transport direction (T) of the relevant substrates after the processing station having the second printing cylinder (118).

4. The machine arrangement according to claim 1 or 2 or 3, **characterized in that** a coating device (08) is arranged in transport direction (T) of the relevant substrates after the processing station having the second printing cylinder (118) and/or after the fourth dryer (09; 124) downstream of this processing station.

5. The machine arrangement according to claim 4, **characterized in that** a further dryer (09) for drying the coated substrates is arranged downstream of the coating device (08).

6. The machine arrangement according to claim 1 or 2 or 3 or 4 or 5, **characterized in that** those retaining elements which are assigned different substrates can respectively be actuated independently and separately from one another.

7. The machine arrangement according to claim 1 or 2 or 3 or 4 or 5 or 6, **characterized in that** the processing stations configured respectively as a primer application device (02; 126) or as a coating device (08) or as a dryer (07; 09; 121; 122; 123; 124) or as a printing device (06; 127) are respectively configured as a module that is able to function independently, wherein each of these modules respectively forms a machine unit mounted in a separate frame.

8. The machine arrangement according to claim 7, **characterized in that** the modules each have a substrate-guide unit (24) and a substrate-processing unit (26), wherein the substrate-guide unit (24) has in each case a transport cylinder arrangement (17; 19) or one or a plurality of transport cylinders (128) or one or a plurality of transfer drums (43; 44) for transporting the substrates.

9. The machine arrangement according to claim 1 or 2 or 3 or 4 or 5 or 6 or 7 or 8, **characterized in that** in each case a substrate-guide unit (24) configured as a pure transport module is or are arranged without a further substrate-processing unit (26) directly after the processing station having the first printing cylinder (22; 117) and/or directly after the processing station having the second printing cylinder (118), wherein each of these transport modules is respectively arranged in an own frame.

10. The machine arrangement according to claim 8 or 9, **characterized in that** the respective substrate-guide units (24) each having a transport cylinder arrangement (17; 19), apart from the two printing cylinders (22; 117; 118) are respectively configured double sized, so that in each case two substrates are arranged or at least can be arranged in tandem at the circumference of the respective transport cylinders (128) or transfer drums (43; 44).

11. The machine arrangement according to claim 1 or 2 or 3 or 4 or 5 or 6 or 7 or 8 or 9 or 10, **characterized in that** the non-impact printing devices (06; 127) each print a plurality of different printing inks, wherein in each case a specific inkjet printing device is provided for each of these printing inks relative to the relevant non-impact printing device (06; 127) and/or that four or five or six or seven non-impact printing devices (06; 127) are arranged in transport direction (T) in tandem.

12. The machine arrangement according to claim 1 or 2 or 3 or 4 or 5 or 6 or 7 or 8 or 9 or 10 or 11, **characterized in that** the dryer (07; 121) for drying the primer in each case applied on the front side of the relevant substrates and/or the dryer (122) for drying the primer in each case applied on the back side of the relevant substrates and/or the dryer (123) for drying the relevant substrates in each case printed on the front side with the non-impact printing device (06) and/or the dryer (124) for drying the relevant substrates in each case printed on the back side with the non-impact printing device (127) and/or the further dryer (09) is or are configured as a dryer drying the relevant primered and/or printed and/or coated substrates in each case by hot air and/or by a radiation with infrared or ultraviolet radiation.

13. The machine arrangement according to claim 1 or 2 or 3 or 4 or 5 or 6 or 7 or 8 or 9 or 10 or 11 or 12, **characterized in that** the retaining elements are respectively configured as grippers.

14. The machine arrangement according to claim 2 or 3 or 4 or 5 or 6 or 7 or 8 or 9 or 10 or 11 or 12 or 13, **characterized in that** the second dryer (09; 123) and/or the fourth dryer (09; 124) are in each case configured as an intermediate dryer.

15. The machine arrangement according to claim 1 or 2 or 3 or 4 or 5 or 6 or 7 or 8 or 9 or 10 or 11 or 12 or 13 or 14, **characterized in that** at least one of the dryers (07; 09; 121; 122; 123; 124) is configured as an LED dryer.

16. The machine arrangement according to claim 1 or 2 or 3 or 4 or 5 or 6 or 7 or 8 or 9 or 10 or 11 or 12 or 13 or 14 or 15, **characterized in that** a resting of the substrates on the lateral surface of the respective printing cylinder (22; 117; 118) is supported by blowing air directed to the relevant lateral surface.

## Revendications

1. Ensemble machine pour le traitement séquentiel de substrats en forme de feuilles, avec plusieurs stations de traitement différentes, dans lequel deux des stations de traitement disposées le long de la voie de transport des substrats présentent respectivement un dispositif d'impression sans impact (06 ; 127) imprimant respectivement les substrats, réalisé respectivement sous la forme d'au moins un dispositif d'impression à jet d'encre, dans lequel les stations de traitement présentant les dispositifs d'impression sans impact (06 ; 127) présentent respectivement un cylindre d'impression (22 ; 117 ; 118), dans lequel le premier cylindre d'impression (22 ; 117) dans la direction de transport (T) des substrats est de taille triple ou quadruple, dans lequel plusieurs dispositifs d'impression à jet d'encre sont disposés les uns à la suite des autres le long d'une partie de la périphérie du premier cylindre d'impression (22 ; 117), dans lequel le deuxième cylindre d'impression (118) dans la direction de transport (T) des substrats est de taille triple ou quadruple, dans lequel plusieurs dispositifs d'impression à jet d'encre sont disposés les uns à la suite des autres le long d'une partie de la périphérie du deuxième cylindre d'impression (22 ; 118), dans lequel pour le prétraitement de la face avant des substrats un premier dispositif d'application de couche de fond (02) et un séchoir (07 ; 121) situé en aval de ce dispositif d'application de couche de fond (02) sont situés en amont de la station de traitement présentant le premier cylindre d'impression (22 ; 117), imprimant la face avant des substrats, **caractérisé en ce qu'**un dispositif de retournement (23) est disposé entre le premier cylindre d'impression (22 ; 117) et le deuxième cylindre d'impression (118), dans lequel tout d'abord un deuxième dispositif d'application de couche de fond (126) traitant la face arrière des substrats et ensuite un troisième séchoir (07 ; 122) sont disposés dans la direction de transport (T) des substrats après le dispositif de retournement (23) et avant la station de traitement présentant le deuxième cylindre d'impression (118), imprimant la face arrière des substrats, dans lequel respectivement plusieurs substrats sont disposés ou tout du moins peuvent être disposés respectivement les uns derrière les autres dans la direction périphérique sur la surface extérieure des cylindres d'impression (22 ; 117 ; 118) respectifs, dans lequel le cylindre d'impression (22 ; 117 ; 118) respectif présente à sa périphérie respectivement au moins autant d'éléments de retenue que trois ou quatre substrats sont disposés ou tout du moins peuvent être disposés à sa périphérie les uns derrière les autres respectivement de manière retenue par une liaison à force et/ou par une coopération de formes.

2. Ensemble machine selon la revendication 1, **caractérisé en ce qu'**un deuxième séchoir (09 ; 123) séchant la face avant imprimée des substrats est disposé dans la direction de transport (T) des substrats après la station de traitement présentant le premier cylindre d'impression (22 ; 117) et avant le dispositif de retournement (23).

3. Ensemble machine selon la revendication 1 ou 2, **caractérisé en ce qu'**un quatrième séchoir (09 ; 124) destiné à sécher la face arrière imprimée des substrats est disposé dans la direction de transport (T) des substrats concernés après la station de traitement présentant le deuxième cylindre d'impression (118).

4. Ensemble machine selon la revendication 1 ou 2 ou 3, **caractérisé en ce qu'**un dispositif de pose de vernis (08) est disposé dans la direction de transport (T) des substrats concernés après la station de traitement présentant le deuxième cylindre d'impression (118) et/ou après le quatrième séchoir (09 ; 124) situé en aval de cette station de traitement.

5. Ensemble machine selon la revendication 4, **caractérisé en ce qu'**un autre séchoir (09) destiné à sécher les substrats sur lesquels un vernis a été déposé est situé en aval du dispositif de pose de vernis (08).

6. Ensemble machine selon la revendication 1 ou 2 ou 3 ou 4 ou 5, **caractérisé en ce que** les éléments de retenue, qui sont associés aux différents substrats, peuvent être actionnés respectivement indépendamment et séparément les uns des autres.

7. Ensemble machine selon la revendication 1 ou 2 ou 3 ou 4 ou 5 ou 6, **caractérisé en ce que** les stations de traitement réalisées respectivement sous la forme d'un dispositif d'application de couche de fond (02 ; 126) ou sous la forme d'un dispositif de pose de vernis (08) ou sous la forme d'un séchoir (07 ; 09 ; 121 ; 122 ; 123 ; 124) ou sous la forme d'un dispositif d'impression (06 ; 127) sont réalisées respectivement sous la forme d'un module fonctionnel de manière autonome, dans lequel chacun de ces modules forme respectivement une unité d'entraînement montée dans un bâti propre.

8. Ensemble machine selon la revendication 7, **caractérisé en ce que** les modules présentent respectivement une unité de guidage de substrat (24) et une unité de traitement de substrat (26), dans lequel l'unité de guidage de substrat (24) pour le transport des substrats présente respectivement un ensemble cylindre de transport (17 ; 19) ou un ou plusieurs cylindres de transport (128) ou un ou plusieurs tambours de transfert (43 ; 44).

9. Ensemble machine selon la revendication 1 ou 2 ou 3 ou 4 ou 5 ou 6 ou 7 ou 8, **caractérisé en ce que** respectivement une unité de guidage de substrat (24) réalisée sous la forme d'un simple module de transport sans autre unité de traitement de substrat (26) est ou sont disposées directement après la station de traitement présentant le premier cylindre d'impression (22 ; 117) et/ou directement après la station de traitement présentant le deuxième cylindre d'impression (118), dans lequel chacun de ces modules de transport est disposé respectivement dans un bâti propre.

10. Ensemble machine selon la revendication 8 ou 9, **caractérisé en ce que** les unités de guidage de substrat (24) respectives présentant respectivement un ensemble cylindre de transport (17 ; 19) à l'exception des deux cylindres d'impression (22 ; 117 ; 118) sont respectivement de taille double, de sorte que respectivement deux substrats sont disposés ou tout du moins peuvent être disposés l'un derrière l'autre à la périphérie des cylindres de transport (128) ou tambours de transfert (43 ; 44) respectifs.

11. Ensemble machine selon la revendication 1 ou 2 ou 3 ou 4 ou 5 ou 6 ou 7 ou 8 ou 9 ou 10, **caractérisé en ce que** les dispositifs d'impression sans impact (06 ; 127) impriment respectivement plusieurs encres d'impression différentes, dans lequel respectivement un dispositif d'impression à jet d'encre défini est prévu pour chacune de ces encres d'impression par rapport au dispositif d'impression sans impact (06 ; 127) concerné et/ou que quatre ou cinq ou six ou sept dispositifs d'impression sans impact (06 ; 127) sont disposés les uns derrière les autres dans la direction de transport (T) des substrats.

12. Ensemble machine selon la revendication 1 ou 2 ou 3 ou 4 ou 5 ou 6 ou 7 ou 8 ou 9 ou 10 ou 11, **caractérisé en ce que** le séchoir (07 ; 121) destiné à sécher la couche de fond appliquée respectivement sur la face avant des substrats concernés et/ou le séchoir (122) destiné à sécher la couche de fond appliquée respectivement sur la face arrière des substrats concernés et/ou le séchoir (123) destiné à sécher les substrats concernés dont la face avant a été imprimée respectivement avec le dispositif d'impression sans impact (06) et/ou le séchoir (124) destiné à sécher les substrats concernés dont la face arrière a été imprimée respectivement avec le dispositif d'impression sans impact (127) et/ou l'autre séchoir (09) est ou sont réalisés sous la forme d'un séchoir séchant les substrats concernés sur lesquels une couche de fond a été appliquée et/ou imprimés et/ou sur lesquels un vernis a été déposé, respectivement par air chaud et/ou par exposition à un rayonnement infrarouge ou ultraviolet.

13. Ensemble machine selon la revendication 1 ou 2 ou 3 ou 4 ou 5 ou 6 ou 7 ou 8 ou 9 ou 10 ou 11 ou 12, **caractérisé en ce que** les éléments de retenue sont réalisés respectivement sous la forme de pinces.

14. Ensemble machine selon la revendication 2 ou 3 ou 4 ou 5 ou 6 ou 7 ou 8 ou 9 ou 10 ou 11 ou 12 ou 13, **caractérisé en ce que** le deuxième séchoir (09 ; 123) et/ou le quatrième séchoir (09 ; 124) sont réalisés respectivement sous la forme d'un séchoir intermédiaire.

15. Ensemble machine selon la revendication 1 ou 2 ou 3 ou 4 ou 5 ou 6 ou 7 ou 8 ou 9 ou 10 ou 11 ou 12 ou 13 ou 14, **caractérisé en ce qu'**au moins un des séchoirs (07 ; 09 ; 121 ; 122 ; 123 ; 124) est réalisé sous la forme d'un séchoir à DEL.

16. Ensemble machine selon la revendication 1 ou 2 ou 3 ou 4 ou 5 ou 6 ou 7 ou 8 ou 9 ou 10 ou 11 ou 12 ou 13 ou 14 ou 15, **caractérisé en ce qu'**une pose des substrats sur la surface extérieure du cylindre d'impression (22 ; 117 ; 118) respectif est facilitée par de l'air de soufflage dirigé sur la surface extérieure concernée.
